# EUROPEAN PATENT SPECIFICATION

(11) **EP 0 827 544 B1**
(45) Date of publication and mention of the grant of the patent: **18.08.2004**
(21) Application number: 96916159.5
(22) Date of filing: 23.05.1996
(51) Int. Cl.: C12N 15/62, C07K 19/00, C07K 14/82, C07K 14/52, C07K 14/78, C07K 14/47, C07K 16/18, C07K 16/28, C12N 1/21, C12N 1/19

(54) **MULTIMERIC PROTEINS**
MULTIMERE PROTEINE
PROTEINES MULTIMERES

(30) Priority: 23.05.1995 EP 95107914
(43) Date of publication of application: 11.03.1998
(73) Proprietor: MorphoSys AG, 82152 Martinsried (DE)
(72) Inventor: PACK, Peter, D-81925 Oberföhring (DE); HOESS, Adolf, D-83629 Weyarn (DE)
(74) Representative: VOSSIUS & PARTNER
(86) International application number: PCT/EP1996/002230
(87) International publication number: WO 1996/037621

(56) References cited:
- WO-A-93/15210
- WO-A-96/13583
- J IMMUNOL METHODS, JAN 27 1995, 178 (2) P201-9, NETHERLANDS, XP000608189 DUBEL S ET AL: "Bifunctional and multimeric complexes of streptavidin fused to single chain antibodies (scFv)."
- HUM ANTIBODIES HYBRIDOMAS, 1995, 6 (3) P93-101, UNITED STATES, XP000607119 KIPRIYANOV SM ET AL: "Single-chain antibody streptavidin fusions: tetrameric bifunctional scFv-complexes with biotin binding activity and enhanced affinity to antigen."
- J MOL BIOL, FEB 10 1995, 246 (1) P28-34, ENGLAND, XP000607470 PACK P ET AL: "Tetravalent miniantibodies with high avidity assembling in Escherichia coli."
- FEBS LETTERS, vol. 341, 1994, AMSTERDAM NL, pages 54-58, XP002017642 EFIMOV VP ET AL: "The thrombospondin-like chains of cartilage oligomeric matrix protein are assembled by a five-stranded alpha-helical bundle between residues 20 and 83" cited in the application
- BIOCHEMISTRY, vol. 33, 1994, EASTON, PA US, pages 8361-8366, XP002017643 LAI CK ET AK: "Crystal structure of recombinant human platelet factor 4" cited in the application
- PROC NATL ACAD SCI U S A, SEP 13 1994, 91 (19) P8974-8, UNITED STATES, XP000608191 SAKAMOTO H ET AL: "Specific sequences from the carboxyl terminus of human p53 gene product form anti-parallel tetramers in solution." cited in the application
- SCIENCE, MAR 10 1995, 267 (5203) P1498-502, UNITED STATES, XP000608194 JEFFREY PD ET AL: "Crystal structure of the tetramerization domain of the p53 tumor suppressor at 1.7 angstroms." cited in the application

## Description

### Field of the invention

The present invention relates to multimeric polypeptides which combine two or more functional domains in a structure which is capable of at least self-trimerization and which can be, for example, readily prepared in recombinant bacteria.

### Background of the Invention

Multivalency is a prerequisite for a variety of macromolecular interactions such as binding of antibodies or lectins to specific targets, ligand recognition, activation or inhibition of receptors as well as cell adhesion.

Carbohydrate binding by lectins such as conglutinin or IgE-binding protein requires multivalency (Hsu et al., 1992, J. Biol. Chem. 267, 14167-14174; Andersen et al., 1992, J. Struct. Biol. 109, 201-27). Compared to the monovalent ligand, the trimeric hepatic lectin (Verrey & Drickamer, 1993, Biochem. J. 292, 149-155) binds the trimeric ligand with an 100 to 1000-fold higher affinity (Lee et al, 1992, Arch. Biochem. Biophys. 299, 129-136).

Multimerization of cell surface receptors as well as of their ligands often define specificity and affinity. The multivalent interactions of CD2 receptors of T-cells with their ligands LFA-3 on the cognate partner are necessary for cell-cell contact and T-cell activation by stimulatory cells (Moingeon et al., 1989, Immunol. Rev. 111, 111-144).

Multivalent binding of heparin to fibroblast growth factor receptors induces receptor dimerization and results in mitogenic signal transduction (Pantoliano et al., 1994, Biochemistry 33, 10229-10248). The concentration-dependent dimerization of the cytokine IL-2 results in high-affinity binding to the IL-2 receptor and formation of the high-affinity complex regulating the subsequent signal transduction (Lother et al., 1992, Growth factors 7, 117-129). Dimerization of CD4 induced by multivalent ligands is necessary to trigger the intracellular tyrosine kinase in T-cells (Langedijk et al., 1993, Thromb.-Res. 71, 47-60). The inhibition of IgE synthesis in allergic diseases requires the injection of at least bivalent anti-IgE immunoglobulins (Stämpfli et al., 1994, Eur. J. Immunol. 24, 2161-2167).

The enhancement of weak monovalent forces by multiplying the number of interactions is increasingly used for protein engineering and therapeutic applications. Synthetic pepides such as the bivalent hirudin-analogs "hirulogs" effectively inhibit clot-bound thrombin (Cannon et al., 1993, Am. J. Cardiol. 71, 778-782), a further optimization of related thrombin inhibitors being achieved by trivalency (Szewczuk et al., 1993, Biochemistry 32, 3396-3404). Multivalent glycopeptides inhibit binding of influenza viruses to their target cells (Unverzagt et al., 1994, Carbohydr. Res. 251, 285-301). The multivalent display of Tyr-Ile-Gly-Ser-Arg peptides strongly inhibits lung metastasis (Nomizu et al., 1993, Cancer-Res. 53, 3459-3461).

The synergistic gain in binding strength (functional affinity or avidity) through multivalent interactions is best characterized for binding of immunoglobulins to their specific antigens. The avidity effect is dependent on the affinity per binding site, flexibility and number of the associated binding sites, number as well as distance of antigens within reach. As a rough estimate, the avidity is the product of affinities per monovalent binding event (Crothers & Metzger, 1972, Immunchemistry 9, 341).

Immunoglobulins such as monoclonal antibodies or heterologously expressed fragments belong to the most promising candidates for "targeted missiles" for screening and combat of tumours and any kind of pathogens in vivo (Colcher et al., 1990, J. Natl. Cancer Inst. 82, 1191-1197).
The heterologous expression of so-called scFv antibody fragments, i. e. the variable domains of antibodies connected by a peptide linker (Bird et al., 1988, Science 242, 423; Huston et al., 1988, Proc. Natl. Acad. Sci. USA 85, 5879) combines several benefits.

The deletion of all constant immunoglobulin domains and the covalent linkage of the two variable domains (Fv) by a peptide linker (Huston et al., 1988, Proc. Natl. Acad. Sci. USA 85, 5879; Bird et al., 1988, Science 242, 423) results in a stable, single-chain immunoglobulin of minimal size with a single, complete binding pocket. The small size of the single-chain Fv fragment (scFv) provides improved expression yields (Skerra & Plückthun, 1991, Protein Eng. 4, 971), proteolytic stability, tissue penetration (Colcher et al., 1990, J. Natl. Cancer Inst. 82, 1191) and decreased nonspecific interactions or immunogenicity compared to complete antibodies. An alternative design links the two variable domains via engineered disulfide bridges at the VL-VH interface (dsFv; Glockshuber et al., 1990, Biochemistry 29, 1362).

The disadvantage of these recombinant fragments, however, is their short in vivo half-life and their inability to form bivalent complexes such as IgG or higher oligomers such as IgM, in which the binding pockets are able to reach two or more distant antigens simultaneously. In medical applications such as tumour targeting, however, the need for efficient tissue penetration and tight binding means that ideally, an immunoglobulin-based structure should combine relatively small size with high affinity/avidity and sufficient half-live (Thomas et al., 1989, Cancer Res. 49, 3290 3296). To regain the avidity of whole antibodies and prolonged half-live in vivo, several techniques were recently developed to access small dimeric and thus bivalent immunoglobulins, although each suffers from substantial disadvantages.

If the interdomain linker between VH and VL in single-chain Fv fragments (Bird et al. , 1988, Science 242, 423-426) is sufficently short, formation of monovalent scFv fragments is disfavoured, resulting in intermolecular association of VH and VL domains of two or more fragments to form scFv-dimers or higher oligomers. In the so-called diabody (Holliger et al., 1993, Proc. Natl. Acad. Sci. USA 90, 6444), the rigid structure and close proximity of the binding sites does not allow flexibility or binding to distant antigens (Perisic et al., 1994, Structure 2, 1217). The formation of stable dimers or higher oligomers is highly dependent on the VH-VL interface of a particular fragment, so that the conversion of an scFv to a diabody is not possible for every given sequence, and the degree of oligomerization is not controllable (Whitlow et al., 1994, Prot. Eng. 7, 1017-1026).

The direct in vitro linkage of two fragments with C-terminal cysteines by oxidation (Carter et al., 1992, Bio/Technology 10, 163) or through chemically attached spacers (Kipriyanov et al, 1994, Mol. Immunol. 31, 1047-1085; Cook & Wood, 1994, J. Immunol. Meth. 171, 227-237) requires laborious isolation and refolding of purified material with poor yields of functional immunoglobulins. Additionally, since mixed disulphide bond formation cannot be excluded, aggregation to unspecified multimers may occur. Non-quantitative disulphide formation further diminishes the overall yield.

The use of amphipathic helices such as bundle helices or leucine zippers C-terminally fused to scFv fragments for dimerization in E.coli (Pack & Plückthun, 1992, Biochemistry 31, 1579-1584) provided access to dimeric, bivalent "mini-antibodies" (PCT/EP93/00082). The antibody fragments assemble exclusively to functional dimers in vivo and can be purified in high yields in a single affinity chromatography step (Pack et al., 1993, Bio/Technology 11, 1271-1277). Based on artificial bundle helices or non-human zipper-sequences, this design of the C-terminally fused "association domains" is potentially hampered by the immunogenicity of such sequences when applied as human therapeutics. Furthermore, bundle helices used as association domains are not able to tetramerize fused antibody fragments, since the association forces of single bundle helices are too weak to stabilize a tetramer (Pack & Plückthun, 1992, Biochemistry 31, 1579-1584).

Modified and therefore "artificial" zipper sequences fused to antibody fragments are able to bring about tetramerization, but incompatibilities of the modified zipper sequence with folding and secretion lead to a considerably diminished yield of functional tetrameric material (Pack et al., 1995, J. Mol. Biol. 246, 28-34) and the complex incorporates only one type of function or functional domain, namely one scFv specificity.

Previously, the CH2-CH3-CH4 domains of immunoglobulin M heavy chains have also been used as carriers of a single functional domain, namely cell surface receptor ligands (PCT Application WO 92/03569). The resulting fusion proteins were expressed in certain mammalian cells as multimers.

For several reasons, the use of CH2-CH3-CH4 domains of immunoglobulin M heavy chains as multimerization devices suffers disadvantages.

First, the expression of such fusion proteins is restricted to certain expression hosts and cellular compartments. The specific glycosylation of constant domains of immunoglobulins is considered to be important for assembly, structure (Rademacher & Dwek, 1984, Prog. Immun. 5, 95-112) function (Burton, 1985, Mol. Immunol. 22, 161-206) and proteolytic stability (Leatherbarrow & Dwek, 1983, FEBS Lett. 164, 227-239) of immunoglobulin constant domains (Matsuda et al., 1990, Mol Immunol. 27, 571-579; Dorai et al., 1991, Hybridoma 10, 211-217). The change or absence of glycosylation patterns, for instance when using a murine instead of a human expression host, results in structural changes and loss of complement activation (Lund et al., 1990, Mol. Immunol. 27, 1145-1153). The correct human glycosylation pattern on immunoglobulin constant domains is only achievable in certain mammalian cell cultures, namely of human origin. A different glycosylation pattern by expression in other than human cells would also increase the immunogenicity in medical applications.

Furthermore, the use of antibody-based association domains of the proposed type suffers from the serious disadvantage of its enormous size of 343 residues per monomer corresponding to three distinct immunoglobulin folding domains (CH2, CH3, CH4) per monomeric fusion protein, with 6 disulphide bridges to be formed (Dorai & Gillies, 1989, Nucleic Acids Res. 17, 6412-6417) or 60 in the decameric, IgM-like complex. Constructs of this type are very unlikely to be producible in bacteria, and there is no report of the functional expression of an immunoglobulin having three correctly folded constant domains in bacteria.

It is well known that the folding efficiency and expression yield of recombinant soluble material depends on the length of the peptide chain, the number of distinct folding domains per chain as well as the number of cis-prolines and correct disulphide bridges to be formed (Jaenicke, 1987, Prog. Biophys. Mol. Biol. 49, 117-237). In procaryotic expression hosts like E. coli, the decrease in yield of larger recombinant proteins is even more pronounced than in yeast or cell culture. As one aspect of this observation, for instance, the proteolysis of recombinant and "foreign" proteins, especially in inter-domain sequences, increases with the number of folding domains per chain. The formation of correct disulphide bridges is a rate-limiting step of folding (Darby et al., 1992, J. Mol. Biol. 224, 905-911) and a prerequisite for a stable immunoglobulin fold (Creighton, 1988, BioEssays 8, 57-53; Skerra & Plückthun, 1991, Prot. Eng. 4, 971-979). In reducing environments such as cytoplasmic compartments, the formation of a disulphide bridge could not be shown (Glockshuber et al., 1992, Biochemistry 31, 1270-1279).

Self-assembly of relatively small fusion proteins, preferably without the need of glycosylation or disulphide bridges in vivo, however, would overcome these general problems mentioned above and result in novel, well expressed complexes.

Bacteria such E. coli provide a fast generation turnover, cheap fermentation, well established molecular biology protocols such as fast access to site-directed mutations. Recently, the display of recombinant proteins on bacteriophages (Greenwood et al., 1991, J. Mol. Biol. 220, 821-827) proved to be a unique tool in combination with random mutagenesis for the generation of large libraries and selection for the most valuable protein mutants. Only small and well expressed multimerization devices, preferably peptidic multimerization devices, would allow the efficient construction of multimeric proteins comprising library-derived recombinant proteins as functional domains.

A further limitation of WO 92/03569 is that it does not teach the preparation of multimers having two or more distinct functional domains per fusion protein. Indeed, W092/03569 only provides for one example of a certain receptor ligand to be assembled in the multimeric proteins when fused to the CH2-CH3-CH4 domains of immunoglobulin M heavy chain and expressed in certain mammalian cells.

None of the prior art can provide substances combining the features of a relatively small size, low immunogenicity, and high yields of functional material with the self-assembly of stable multimeric complexes larger than dimers having at least two distinct functions.

The additional fusion of a second functional domain C-terminal to a peptidic, self-assembling multimerization device via a second flexible linker significantly broadens the range of applications of multimeric proteins. Self-assembly of a bifunctional fusion protein with the help of a "central" multimerization device (domain1-linker1-device-linker2-domain2) would result in a completely novel recombinant complex having two functions in preferably multiple copies in one complex.

A wide range of combinations of two different functional domains is now within reach. The assembly of two or more different antibody fragments to multivalent complexes with valencies equal to/or more than 3 per specificity as well as combination of unrelated functional domains such as enzymes, toxins, cytokines, kinases, phosphatases, lectins, peptide hormones, cell adhesion proteins such as integrins, metal-binding domains, peptidic vaccines, bioactive peptides or soluble cell surface proteins such as the CD molecules of leucocytes or parts thereof in one multimeric complex are of considerable interest for diagnostic and therapeutic applications.

The drawbacks associated with the prior art are overcome by the present invention, which is concerned with the preparation of multifunctional proteins which show the following features:
(i) they can be prepared using standard recombinant microorganisms, even though the molecular weight of the assembled protein exceeds that of proteins commonly expressed in bacteria,
(ii) low immunogenicity in humans
(iii) exists as multimers of valency equal to/or greater than three
(iv) provide proteins carrying two or more functional domains in a single multimeric structure.

The present invention surprisingly provides small peptidic multimerization devices which support two functional domains, which may be distinct from each other, as N- and C-terminal fusions, with all three subunits folding into functional forms, and which can be produced without the need for refolding, for instance, in recombinant bacteria.

Within a multimeric protein of this invention, which can be encoded and translated as a single, contiguous monomeric polypetide chain (domainl-linkerl-multimerization device-linker2-domain2) with posttranslational self-assembly, such a peptidic multimerization device, however, must not greatly interfere with the folding and function of the N- and C-terminally fused domains, and it must self-associate in the steric context of the fused domains at both termini, even when the functional domains are of much larger size than the peptidic multimerization device. Peptidic multimerization devices of this invention provide for these properties.

It is well known in the art that not all kinds of polypeptides, i.e. functional domains can be produced in sufficient amounts and native fold in every expression host. This is due to differences in the folding and secretion environment, post-translational modifications such as glycosylation patterns and protease activities. However, the present invention also provides a solution to overcome these prior art problems. Namely, in these cases, a combined use of different expression hosts and/or in vitro synthesis has to be used to create multimeric proteins with a central, peptidic multimerization device of this invention.

A certain functional domain, for instance, may require folding in the cytoplasm of a prokaryotic organism with subsequent refolding, but the second functional domain, with which the first functional domain should be combined does not properly fold in the cytoplasm of a prokaryotic organism but would need secretion in a mammalian host with subsequent glycosylation for a native fold.

Certain functional domains or parts therof can be produced in vitro in cell-free translation systems (Nishimura et al., 1995, J. Fermentation and Bioengineering 80, 403-405) or as completely synthetic polypeptides (Fitzgerald et al., 1995, J. American Chemical Society 117, 11075-11080). Polypeptides with more than 100 amino acid residues, for instance, are currently synthesized using Fmoc chemistry on solid phase (Roggero et al., 1995, Molecular Immunology 32, 1301-1309).

Using in vitro synthesis, either a part or a complete multimeric protein of this invention can be synthesized and subsequently coupled, e.g. chemically or enzymatically in vitro (e.g. Harris et al., 1973, FEBS Lett. 29, 189-192) or by oxidation of additional, free cysteins (Carter et al., 1992, Bio/Technology 10, 163) to the remaining parts, which are separately produced in a different expression system or expression host. For example, the multimerization core may be cheaply synthesized in E. coli whereas the linkers and functional domains may be produced in different expression systems such as CHO cells, or bioactive peptides as functional domains by in vitro synthesis.

The benefits of a peptidic multimerization device of this invention can be demonstrated using the example of scFv fragments as N- and/or C-terminally fused functional domains. The multimeric protein combines greatly improved properties such as multivalent binding and bispecificity with prolonged half-live in vivo making it therefore particularly suitable for a variety of therapeutic applications. Fullfilling the needs of small size for tissue penetration, monomeric scFv fragments do not show avidity effects and are rapidly excreted, mainly via the kidneys. Their half-live t(1/2 alpha) is around 10-times shorter than that of a whole, bivalent antibody and 2 to 3-times shorter than that of a dimeric scFv fragment (Haunschild et al., 1995, Antibody, Immunoconjugates and Radiopharmaceuticals 8, 111-127), diminishing the chance to find the specific antigen and bind thereto. Multimeric scFv fragments are expected to have an considerably higher half-live and circulation time in vivo than monomeric or dimeric scFv fragments. Additionally, they can bind multivalently and can combine two specificities in one multimeric complex (the N-terminal scFv fragment as a functional domain may have a different specificity than the C-terminal functional domain).

The present invention provides for multimeric proteins comprising scFv fragments as N-and C-terminal functional domains which, for instance, combine two distinct scFv fragments (scFv1-linker1-device-linker2-scFv2)_{>2}. This entirely new design of multivalent & bispecific immunoglobulins is of enourmous interest for diagnostic and therapeutic purposes, since it enables, for instance, multivalent targeting of tumor tissue (specificity scFv1) and the recruitment of cytotoxic T-cells (specificity scFv2) to the tumor (Perez et al., 1985, Science 316, 354-356).

Furthermore, the present invention generally provides for heterotetramers that combine a variety of functions in one molecule. For example, the heterotetramerization may be a consequence of the association of structurally different multimerization devices. Alternatively, they may be the result of differnt functional domains fused N- or C-terminally (via the same or different linkers) to said devices as is detailed hereinbelow. Thus, the peptidic multimerization domain of this invention can be based on parts of natural proteins with the ability to heteromultimerize, such as the the histone (H3/H4)₂ heterotetramer or the (TAF_{II}42/TAF_{II}62)₂ heterotetramer (Xie et al., 1996, Nature 380, 316-322), which both require less than 80 residues per subunit to heterotetramerize. Used as multimerization devices of this invention, 4 different functional domains (two domains N-terminally and two C-terminally fused via flexible linkers to the multimerization devices) can in principle self-assemble to a multimeric protein, in which 4 distinct functional domains appear twice.

It was surprisingly shown that the multimerization device fused to the linker and functional domain sequences as described in accordance with the present invention allows independent folding (Mack et al., Proc. Natl. Acad. Sci. USA 92, 7021-7025 (1995) and is essentially resistant to proteolytic attack which is not true for prior art constructs that employ a certain linker length. The linkers used in accordance with the present invention are preferably not larger than 15 amino acids.

The present invention also provides for a multimeric protein wherein the first functional domains are different for each monomer and/or the second functional domains are different for each monomer. I this embodiment, the present invention also provides for the possibilities that the first and second functional domain(s) are the same or different.

Additionally, the present application provides for the possibility that the first and/or second functional domain(s) is/are fused to at least one further (functional) domain. Such constructs may advantageously comprise tags as functional domains at their N- or C-termini which may be used for purification purposes.

The present invention generally also provides for multimeric proteins of synthetic, semisynthetic or recombinant origin, wherein at appropriate locations cysteine residues are placed that are capable of forming cystine bridges within the multimerization device. Such cystine bridges confer a further enhanced stability on such multimeric proteins. The person skilled in the art is well aware of how such cysteines should be introduced into the multimerization devices and where they should be placed. For example, by appropriatly manipulating the DNA sequence of the invention, certain amino acid codons may be changed into cystein codons. Accordingly, thus modified DNA sequences are also comprised by the present invention.

The present invention also provides multimeric proteins and DNA sequences encoding the monomeric parts thereof, respectively, comprising appropriately designed additional cysteines as part of the multimerization device or the linker(s) to allow for intermolecular covalent linkage to additional functional domains, which are separately produced in vivo or in vitro and, for example, coupled to the multimeric protein via oxidation of a free cystein to result in a covalent disulphide bridge.

The present invention also provides for multimerization devices, which are derived from libraries of randomized DNA sequences (EP 95 11 3021.0-2110 and EP-A 0 614 989). Such partially or fully randomized DNA sequences can be fused, for instance, to part of the gene III (gIII) of filamentous phage in an appropriate phagemid. After transformation of the phagemids into E. coli, gIII proteins can be produced as a fusion with a peptide encoded by one member of the library. After infection with helper phage, new phages are produced displaying the peptide of interest and incorporating the phagemid encoding for the displayed peptide. New multimerization devices can be identified by the interaction of the displayed peptide with a target peptide, which is, for instance, immobilized on a solid surface or fused to an infection mediating particle ("IMP").

### Detailed description of the invention

The object of this invention is a family of multimeric proteins which combine two or more functional domains. The multifunctional proteins of the present invention are based on small preferably peptidic multimerization devices which at least trimerize, and which can be N- and C-terminally fused to functional domains to form novel multimeric and multifunctional polypeptide complexes. The invention also provides DNA sequences, vectors, encoding said multifunctional proteins as well as methods for their production in vitro and in vivo. The invention further relates to gene cassettes as well as to pharmaceutical and diagnostical compositions comprising the protein of the invention.

Specifically, the present invention provides DNA sequences which encode:
(i) a first functional domain,
(ii) a first linker sequence,
(iii) a multimerization device of preferably 30-80, and not more than 110 amino acids in length, which is capable of self-assembly to a trimer or higher order oligomer, and which is preferably of human composition,
(iv) a second linker sequence, and
(v) a second functional domain.

In this context, the term "functional domain" refers to an oligo- or polypeptide which has one or more functions, such as binding to a defined target substance, catalyzing reaction of a defined substrate, inhibiting the action of an enzyme, binding or blocking a receptor binding site or binding to a metal ion.

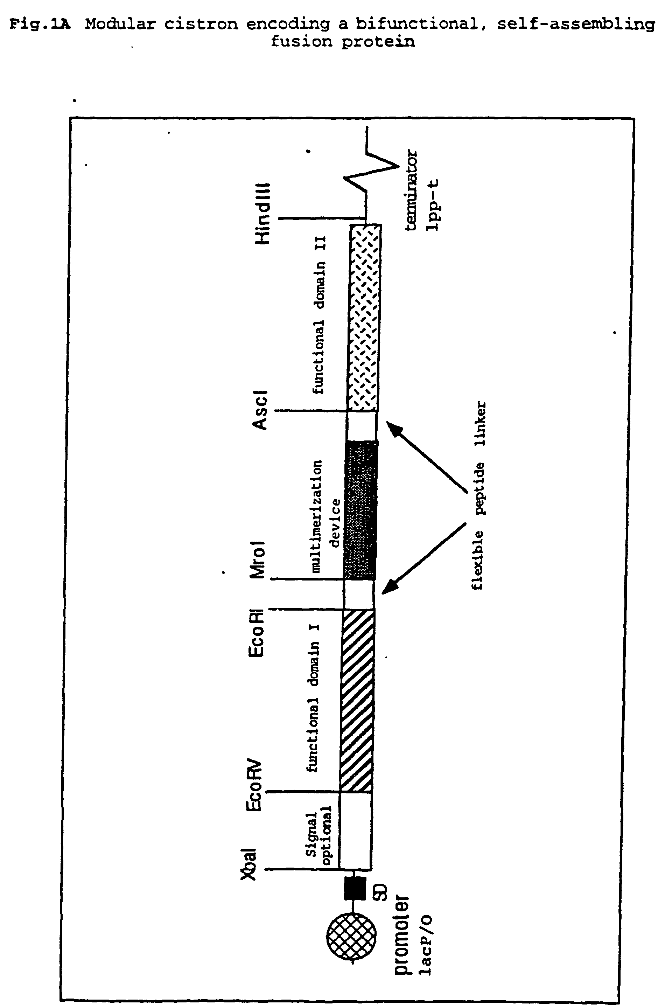

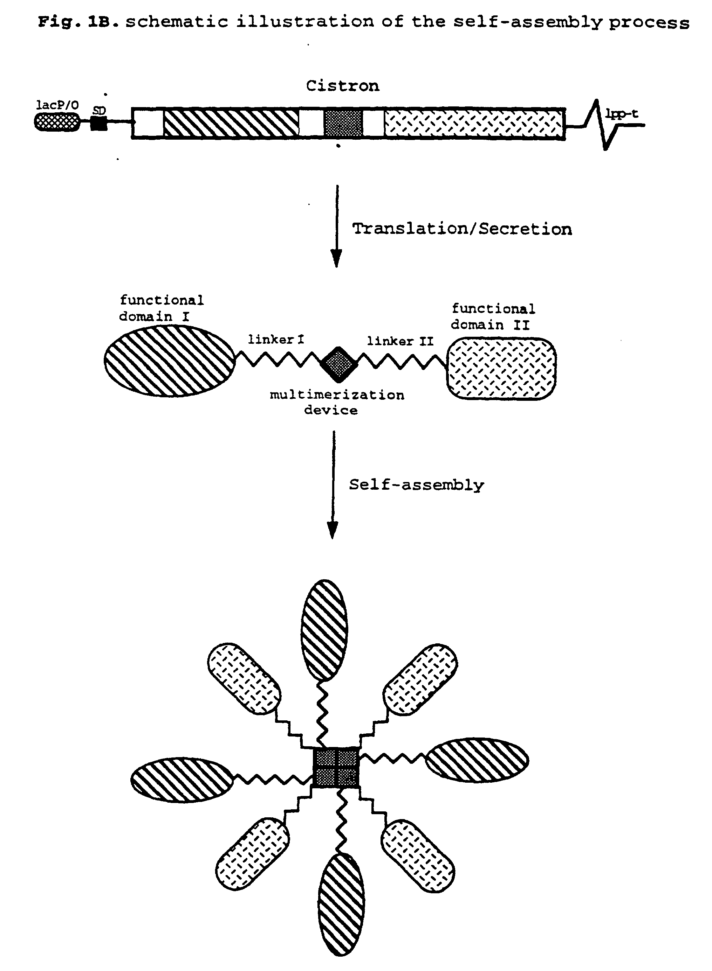

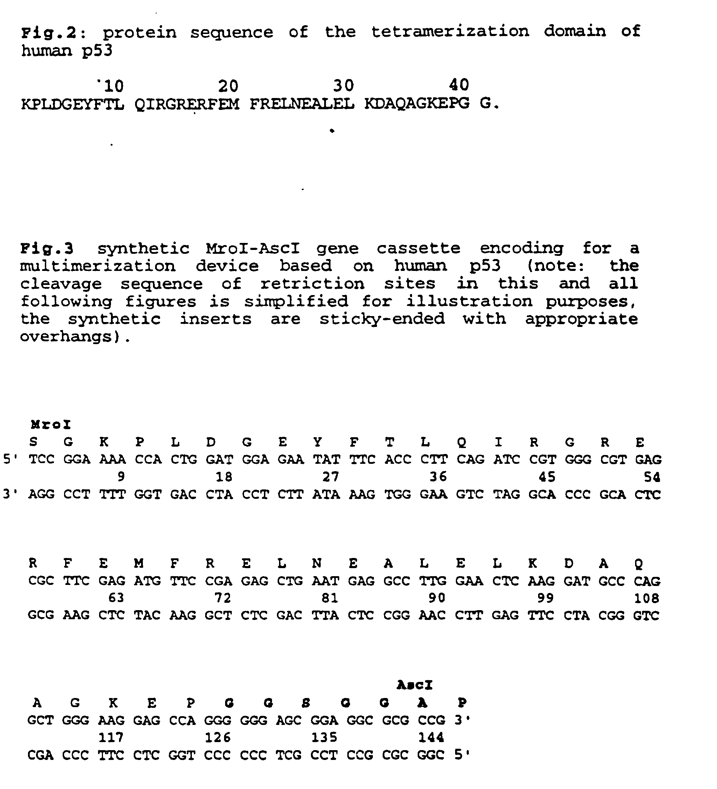

The items (i) to (v) mentioned above are preferably contiguous, and comprise a single open reading frame, allowing controllable expression of a single multifunctional protein from an appropriate vector. The illustration of the fusion principle of such a DNA sequence encoding a bifunctional, self-associating protein is shown in Fig. 1A.

The term 'linker sequence' is well known in the art. The linker used is not crucial for the present invention. Thus, in one construct, one and the same or different sequences can be used for the first and the second linker sequence.

A particular preferred embodiment of this invention is the use of a peptidic multimerization device based on a modified C-terminus of human p53 protein (Clore et al., 1994, Science 265, 386-391; Sakamoto et al., 1994, Proc. Natl. Acad. Sci. USA 91, 8974-8978; Soussi et al., 1990, Oncogene 5, 945-952).

The gene product of the human p53 tumor suppressor gene (Hollstein et al., 1991, Science 253, 49-53) contains distinct modular sequences for trans-activation (Unger et al., 1992, EMBO J. 11, 1383-1386), DNA-binding (Erlandson & Verdine, 1994, Chemistry & Biology 1, 79-84) and tetramerization (Clore et al., 1994, Science 265, 386-391; Sakamoto et al., Proc. Natl. Acad. Sci. USA 91, 8974-8978). The sequence reponsible for tetramerization of the p53 (residues 319-360; Fig.2) can be separately expressed in E. coli (Studier et al., 1990, Methods Enzymol. 185, 60-64) resulting in a stable, symmetric 20 kD tetramer, containing helical as well as beta-strand secondary structures (Jeffrey et al., 1995, Science 267, 1498-1502).

The particular preferred p53-based multimerization device surprisingly allows self-assembly to a tetrameric complex with two different functional domains fused to its termini (Examples 1 - 3), where each of the fused functional domains can be more than eight times larger than the device (Example 2). Suprisingly and contrary to what the person skilled in the art would have expected, the human p53-based peptidic multimerization device has, in several cases, not greatly or significantly interfered with secretion, independent folding and function of the domains fused via flexible linkers, since the properties of both functional domains can be detected in the tetrameric complex directly isolated from the cell extract (Example 1). The small size and human origin of the p53 based peptidic multimerization device enables a significantly reduced immunogenicity in human compared to artificial or non-human self-associating sequences.

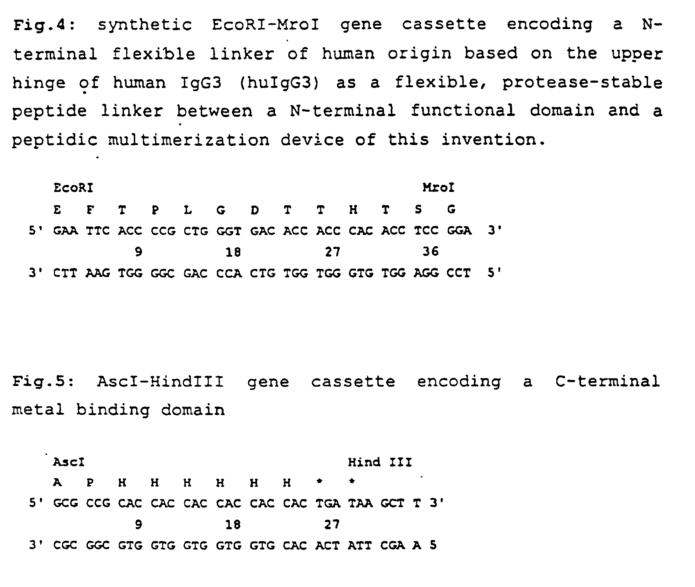

In a further preferred embodiment, the engineered peptidic multimerization device comprises self-associating, modified human platelet factor 4 (PF4; Zhang et al., 1994, Biochemistry 33, 8361-8366). PF4 (Fig. 4) is an abundant human serum protein of 101 residues secreted by platelets. It can be heterologously expressed in E. coli and self-assembles into tetramers with antiparallel beta-sheet like structures (Zhang et al., 1994, Biochemistry 33, 8361-8366).

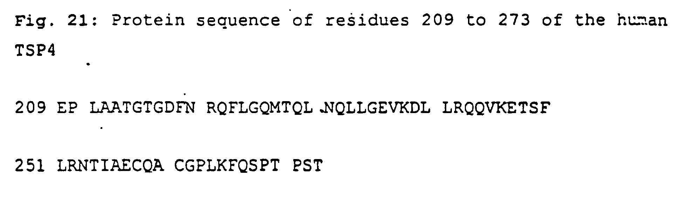

The extracellular human protein TSP4, forming the basis of a further preferred embodiment of this invention, is a member of the thrombospondin family, which modulates the attachment of a variety of cells (Lawler et al., 1993, J. Cell. Biol. 120, 1059-1067). Residues 209 to 273 of human TSP4 (Fig. 21) show a high degree of homology to residues 20 to 83 of the cartilage oligomeric protein (CMP or COMP). This homologous region is responsible for pentamerization of COMP and TSP4 (Efimov et al., 1994, FEBS letters 341, 54-58; Jenkins et al., 1990, J. Biol. Chem. 265, 19624-19631). Pentamerization of functional domains can be achieved by C- or N-terminal fusion to a TSP4-based peptidic multimerization device according to Examples 1 and 2.

In a preferred embodiment of the invention, the multimerization device is derived from a randomized DNA library in a suitable host, from which DNA members are identified by their ability to encode multimerizing peptides. Means and methods for such identification are well known to the person skilled in the art; see, e.g., EP 95 11 3021.0, or EP-A 0 614 989, which are incorporated herein by reference.

The present invention also provides for proteins, preferably fusion proteins, multimeric proteins consisting e.g. of such fusion proteins as well as methods of producing multimeric proteins of this invention. Such a method relates to the production of a protein comprising
a) a first functional domain,
b) a first linker sequence,
c) a preferably peptidic multimerization device of not more than 110 and preferably 30-80 amino acid residues in length, which is capable of self-assembly to a trimer or higher order oligomer, and which is of predominantly human composition,
d) a second linker sequence, and
e) a second functional domain,
wherein at least one of a) to e) is
i) produced recombinantly, and, if all of a) to e) are produced recombinantly, at least two of the constituents are produced by different host cells; and/or
ii) produced synthetically; and/or
iii) produced semisynthetically; and/or
iv) produced by in-vitro translation; and
wherein at least two of a) to e) are combined by enzymatic and/or chemical coupling thereby giving rise to the complete protein, and preferably to the complete multimeric protein.

For therapeutic purposes, it is often desirable that proteinaceous substances display the minimum possible immunogenicity. Accordingly, the present invention provides for multimeric polypeptides as described above in which at least one of said amino acid sequences, functional domains, or further (poly)peptides is of human origin. For example, the extracellular origin of the peptidic multimerization device based on parts of human PF4 or COMP is anticipated to provide for low immunogenicity in humans.

In a further preferred embodiment of this invention, the peptidic multimerization device comprises parts of the TATA Box binding protein associated factors (TAFIIs), preferably of human origin. Drosophila dTAFII42 and dTAFII62 are able to form hetero-tetrameric complexes resembling the (H3/H4)2 hetero-tetrameric core of the chicken histone octamer (Xie et al., 1996, Nature 380, 316-322). The human homologues to dTAFII42 and dTAFII62 are hTAFII31 (Hisatake et al., 1995, Proc. Natl. Acad. Sci. USA 92, 8195-8199) and hTAFII80 (Histake et al., 1995, Proc. Natl. Acad. Sci. USA 92, 8195-8199; Weinzierl et al., 1993, EMBO J. 12, 5303-5309). Residues 13 to 87 of the human hTAFII31 and residues 10 to 82 of the human hTAFII80 can be used as a heterotetramerization device of this invention. Different nomenclature may exist for identical hTAFII-proteins, such as hTAFII80 is also known as hTAFII70 (Hisatake et al., 1995, Proc. Natl. Acad. Sci. USA 92, 8195-8199).

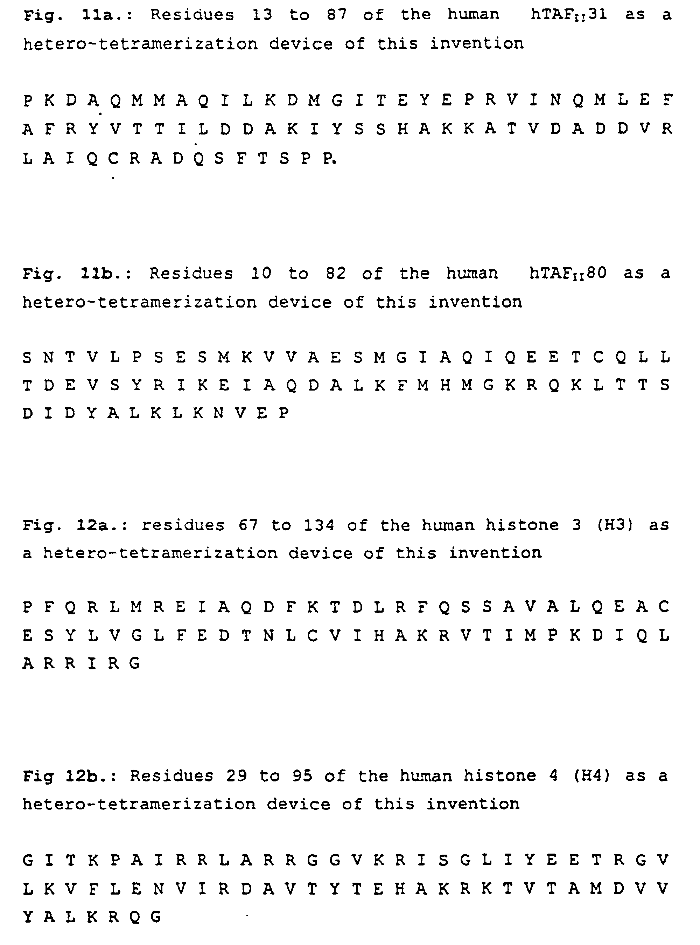

In a further preferred embodiment of this invention, the peptidic multimerization device comprises parts of the histone H3 and H4 proteins, preferably residues 67 to 134 of human histone 3 (H3) and residues 29 to 95 of the human histone 4 (H4) (Fig 12a, b). The human H3 and H4 are highly homologous to the chicken H3 and H4, which resemble the structurally similar dTAFII42/dTAFII62 hetero-tetramer (Xie et al., 1996, Nature 380, 316-322).

In order to provide sufficient distance and flexibility between the peptidic multimerization device and fused functional domains, the peptidic multimerization device is linked via preferably hydrophilic, preferably flexible but protease-resistant polypeptide linkers to the functional domains. In a preferred embodiment of this invention, one or both of the linkers comprise protease-stable inter-domain linkers of human proteins (Argos, 1990, J. Mol. Biol. 211, 943-958), which provide sufficient flexibility and hydrophilicity. In a further preferred embodiment, the linker sequence is rich in the amino acid residues serine, threonine, glycine and proline, which provide an extended structure, rotational freedom, hydrophilicity as well as stability against proteases (Argos, 1990, J. Mol. Biol. 211, 943-958). In a particular preferred embodiment of the invention, the linker is a human antibody hinge sequence, particularly the TPLGTTTHT sequence derived from the upper hinge region of the human IgG3 antibody (Fig.4). In this context, the term antibody hinge sequence refers to an oligopeptide which links CH1 and CH2 domains and is responsible for flexibility, rotational freedom and long range of the Fab arm to bind simultaneously to distant antigens (Dangl et al., 1988, EMBO J. 7, 1989-1994)

The present invention provides for many different types of functional domains to be multimerized. Thus, they may
a) bind to a defined target substance, or
b) catalyze reaction of a defined substrate, or
c) inhibit the action of an enzyme, or
d) bind or block a receptor binding side, or
e) bind to a metal ion.
Preferred are also cases in which the sequences of at least one of the C- or N-terminal functional domains fused to the multimerization device comprise at least a part of a member of the immunoglobulin superfamily. In a particularly preferred embodiment, said functional domain is an antibody scFv fragment. This aspect of the invention provides multivalent antibody-like molecules, which can be of utility when simultaneous multivalent interaction with antigen is a prerequisite of recognition and stable binding to the specific antigen. This is the case, for instance, with carbohydrate antigens on a cell surface, against which individual antibody binding sites show poor affinity.

Also preferred are cases in which one or more functional domains fused to the peptidic multimerization device possess biological activity other than that of a member of the immunoglobulin superfamily. By way of example, the present invention provides for the targeted multimerization of enzymes, toxins, cytokines, kinases, phosphatases, lectins, peptide hormones, cell adhesion proteins such as integrins, metal-binding domains, peptidic vaccines, bioactive peptides or soluble cell surface proteins such as the CD molecules of leucocytes or parts thereof in one multimeric complex.

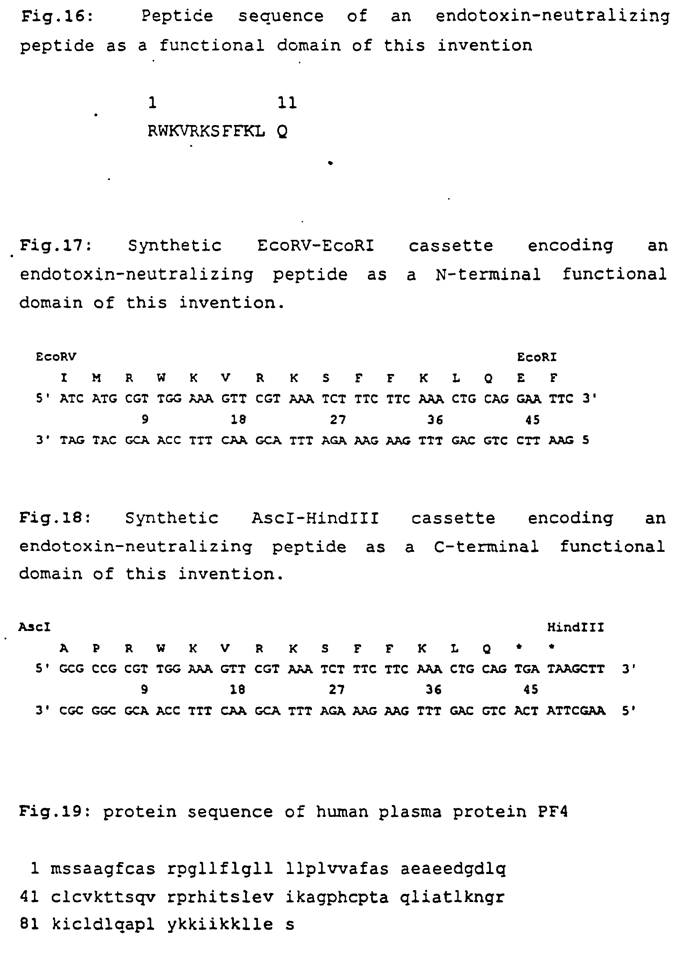

A preferred embodiment of this invention relates to constructs where the functional domains are bioactive peptides. Certain peptides derived from amphipathic loop structures of LPS-binding proteins (Hoess et al., 1993, EMBO J. 12, 3351-3356) are able to neutralize endotoxin. LPS often occurs in multimeric forms and it is therefore desirable to have access to multivalent forms of short (10-15 residues) LPS-binding peptides to ensure effective binding and neutralization of endotoxin. The present invention provides a method to express and multimerize several short peptides (Fig. 16) fused to the multimerization device. The peptides can be fused either to the N- or to the C-terminus (Fig. 17, 18) of the assembly domain via the peptide linkers. A fusion to both termini, e.g., to a p53 based multimerization device, results in a self-assembling octavalent complex of bioactive peptides. Alternatively, a combination with other functional domains can be achieved by their fusion to the opposite terminus of the peptidic multimerization device, resulting in a self-assembling complex displaying four bioactive peptides and four further functional domains.

The invention enables complex multimeric and multifunctional polypeptides to be constructed. By way of example, the second functional domain fused to the peptidic multimerization device can be taken from the list of cytokines, toxins, enzymes, kinases, phosphatases, lectins, peptide hormones, cell adhesion proteins such as integrins, metal-binding domains, purification devices, in particular peptides which are able to bind to an independent binding entity, peptidic vaccines, bioactive peptides, preferably of 5 to 15 amino acid residues, soluble cell surface proteins such as the CD molecules of leucocytes, DNA binding domains, transcription factors and growth factors or parts thereof.

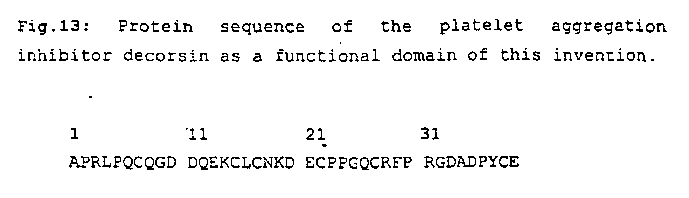

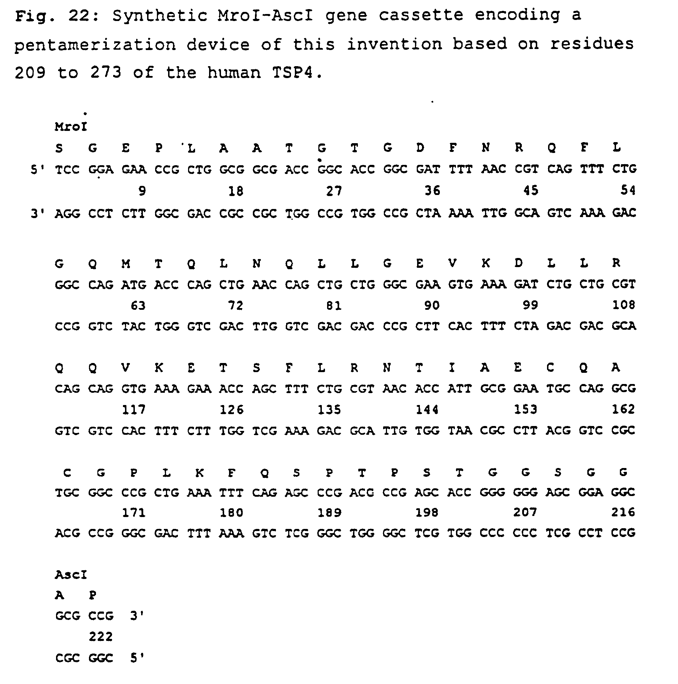

A preferred embodiment of this invention is the use of decorsin as a functional domain. Decorsin is a 39 residue protein of the leech Macrobdella decora (Fig.13). It acts as a potent antagonist of the platelet glycoprotein IIb-IIIa (Seymour et al., 1990, J.Biol.Chem. 265, 10143-10147). According to example 2, the decorsin can be fused C- or N-terminally to the peptidic multimerization device (Fig. 3, 11, 12, 20, 22) and expressed in a suitable host. In arterial thrombotic diseases, the translated self-assembling multivalent decorsin complex may act as a powerful antithrombotic agent. Optionally, the N-terminal fusion of an anti-fibrin antibody fragment to a peptidic multimerization device, which carries a C-terminally fused decorsin, may result in targeting of the multimeric complex to blood clots.

The present invention provides for a standarized system of gene cassettes encoding functional domains, linkers and peptidic multimerization devices. Modular gene cassettes can easily be combined via unique restriction enzymes to a cistron (Fig. 1A). The cistron encodes for a self-associating protein, in which each monomeric subunit consists of an optional signal sequence for secretion, a first functional domain, a first linker sequence, an assembly domain of 30-110 amino acids in length, a second linker sequence and a second functional domain.

In a preferred embodiment said gene cassettes are directly cloned as a cistron into standard expression vectors enabling replication, under a regulatable promoter, translation and optionally secretion of the cistron-encoded protein. Host cells transformed with at least one vector or vector cassette of the invention can be used for the preparation of said multimeric polypeptides.

In a further preferred embodiment said host cell is a mammalian, preferably human, yeast plant or bacterial preferably an E.coli cell.

The invention further provides for methods for the production of said multifunctional proteins, which methods comprise culturing a host cell of the invention in a suitable medium, and recovering said multimeric polypeptide produced by said host cell.

The invention further provides for methods for the separate production of parts of the multimeric protein such as the peptidic multimerization domain or/and one or both functional domains, and multimeric proteins and monomers thereof produced by said method which methods comprise at least one of peptide synthesis, in vitro translation, expression in hosts other than the hosts in which the remaining part of the multimeric protein is produced (e.g. by species, cell ionic or strain difference), and refolding with chemical coupling, e.g. subsequent or enzymatic, to the separately produced remaining part of the functional protein to result in a polypeptide that assembles to a multimeric protein of this invention.

The invention further provides for the introduction of appropriately placed additional cysteins to allow covalent intermolecular linkage of the multimerization device during or after self-assembly.

The invention further provides for the introduction of appropriately placed additional cysteins to allow linkage of further functional domains, for example, by oxidation to intermolecular disulfide bridges.

In further preferred embodiments, the present invention provides for pharmaceutical and diagnostic compositions comprising the multimeric polypeptides described above, said pharmaceutical compositions optionally comprising a pharmaceutically acceptable carrier.

Finally, the invention provides for a kit comprising one or more vector cassettes useful in the preparation of said multimeric polypeptides.

The invention is now illustrated by reference to the following examples, which are provided for the purposes of illustration only and are not intended to limit the scope of the invention.

### EXAMPLES

**Example 1:** Formation of tetrameric complex incorporating four copies anti-Le^{Y} scFv antibody fragments and four copies of a metal-binding domain by fusion of the two different functional domains to the 5'- and 3'-termini of a small tetramerization device based on human p53 via flexible linkers.

The peptidic multimerization device of this example is based on a modified C-terminus of human p53 (Soussi et al., 1990, Oncogene 5, 945-952; Clore et al., 1994, Science 265, 386-391; Fig. 2). A gene cassette (Fig. 3) synthesized by recursive PCR (Prodromou & Pearl, 1992, Protein Eng. 5, 827-829) encodes residues 319-360 of human p53 flanked by a SG-peptide incorporating an in-frame unique 5' restriction site (MroI) and a short C-terminal GGSGGAP linker incorporating an in-frame AscI restriction site at the 3'- terminus of the gene cassette encoding the peptidic multimerization device. The synthetic modular MroI-AscI gene cassette is 5' ligated to an EcoRI-MroI cassette encoding the human IgG3 upper hinge (Dangl et al., 1988, EMBO J. 7, 1989-1994; Fig. 4). The ligation product, a EcoRI-HindIII gene cassette encoding the tetramerization device, can be used for 5' (N-terminal) as well as 3' (C-terminal) fusions to genes encoding recombinant proteins or functional domains such as cytokines, toxins, immunoglobulins, enzymes, kinases, phosphatases, lectins, peptide hormones, cell adhesion proteins such as integrins, metal-binding domains, purification devices, in particular peptides which are able to bind to an independent binding entity, peptidic vaccines, bioactive peptides, preferably of 5 to 15 amino acid residues, soluble cell surface proteins such as the CD molecules of leucocytes, DNA binding domains, transcription factors and growth factors (see further examples).

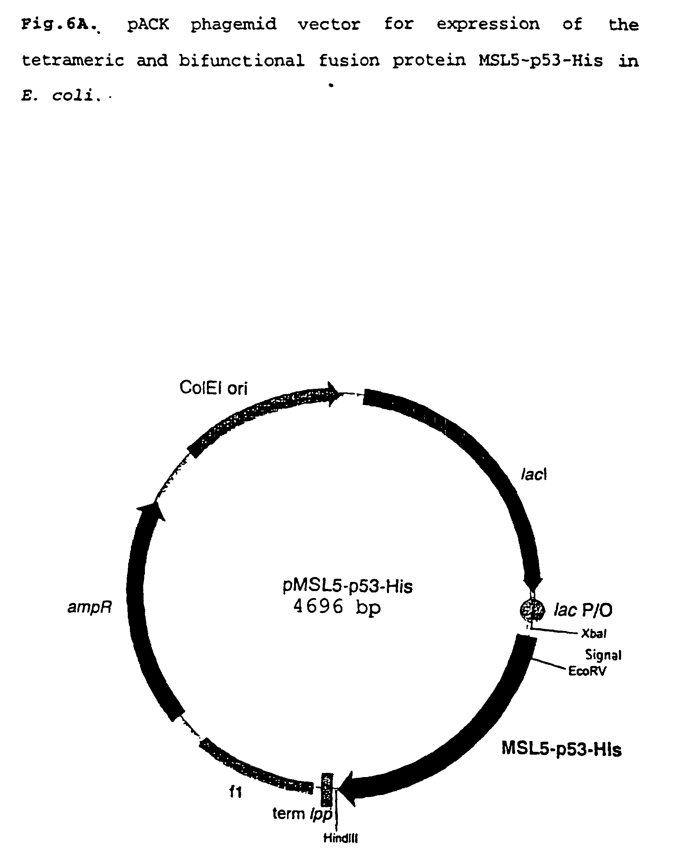

A cistron of the fusion protein of this example is constructed by the ligation of the following modular gene cassettes (5' -> 3') to a cistron und under a lac promoter/operator system in an appropriate E. coli expression plasmid (Fig. 6a, Ge et al., 1995, Antibody Engineering 2nd edition, C.A.K. Borrebaeck, ed. Oxford University Press, pp 229-266, Glockshuber et al., 1990, Biochemistry 29, 1362-1367; Skerra et al., 1991, Bio/Technology 9, 273-278):
- a XbaI-EcoRV cassette encoding an ompA signal sequence
- an EcoRV-EcoRI cassette encoding a LeY carbohydrate-binding scFv antibody fragment
- an EcoRI-MroI AscI gene cassette encoding a flexible upper hinge of human IgG3 (Fig. 4)
- a MroI-AscI encoding a peptidic multimerization device derived from the human IgG3 resuidues human p53 and a flexible GGSGGAP linker incorporating the in-frame AscI-site (Fig. 3)
- a AscI-HindIII gene cassette encoding a functional domain for purification purposes which complexes metal ions.

The EcoRV-EcoRI cassette encoding the LeY carbohydrate-binding scFv antibody fragment MSL-5 was constructed by PCR amplification of the V-region cDNA of a hybridoma (Bradbury et al., 1995, Antibody Engineering 2nd edition, C.A.K. Borrebaeck, ed. Oxford University Press, pp 295-361) secreting anti-LeY antibodies. Appropriate PCR primers were used to introduce the EcoRV and EcoRI restriction sites and to assemble the scFv fragment with a 15 residue (Gly₄Ser)₃ interdomain linker in a VL to VH polarity (Ge et al., 1995, Antibody Engineering 2nd edition, C.A.K. Borrebaeck, ed. Oxford University Press, pp 229-266).

After transformation of the expression plasmid into an E. coli JM83 strain and induction of the lac promotor with IPTG at room temperature in shaking cultures, the translated fusion protein (MSLS-P53-His) is secreted into the periplasm and self-assembles into a tetrameric protein of this invention with four scFv fragments and four metal binding domains in one complex ("multibody"). In addition to applications such as incorporation of radioactive metal ions into a tetravalent anti-tumour complex, the metal binding function of the C-terminal domain can also be used for purification purposes. For example, the complex is isolated by immobilized metal ion chromatography IMAC (Lindner et al., 1992, Methods: a companion to Methods in Enzymology 4, 41-56; Fig. 5). The metal binding property of the tetrameric complex is demonstrated by the success of the purification procedure.

The peptidic multimerization device is compatible with secretion and folding of the anti Le^{Y}-binding scFv fragment, so that no reduction in functional yields (compared to the monovalent scFv) is seen. Unexpectedly, the tetrameric and thus tetravalent fusion protein showed an improved expression behaviour compared to the monomeric scFv in this example, probably due to a reduced toxicity.

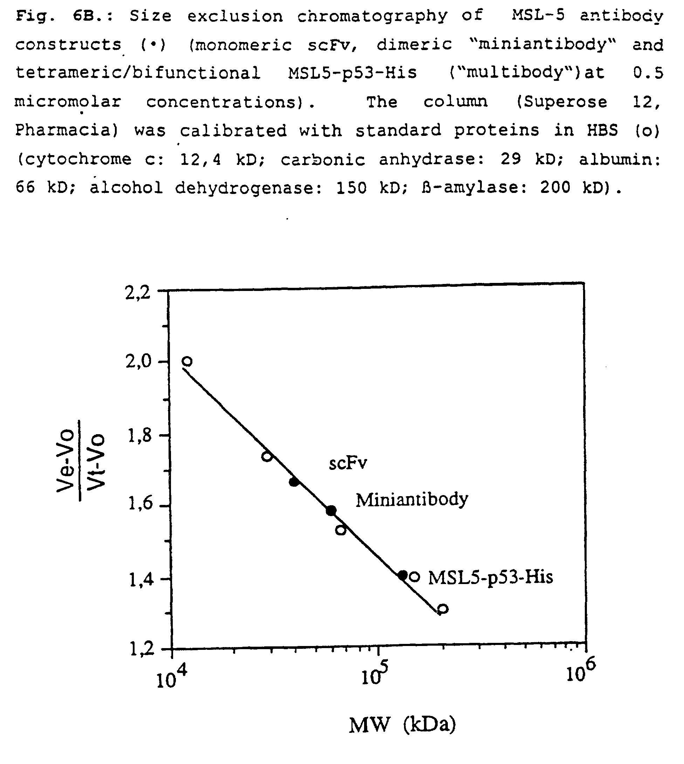

The tetramerization of the bifunctional fusion protein to a stable complex is shown by size exlusion at 0.5 micromolar concentration (Fig. 6B).

The characterization of binding kinetics to a Le^{Y}-coated biosensor surface was performed by surface plasmon resonance on a BIAcore (Pharmacia; Pack et al., 1995, J. Mol. Biol. 246, 28-34). All measurements were carried out in HBS buffer (Pharmacia) with a flow rate of 5 µl/min at 25°C using one CM5 sensor cell of a biosensor machine (BIAcore, Pharmacia). LeY-BSA was covalently immobilized on the dextran matrix of a CM5 sensor chip using the standard amine immobilization procedure. Following activation of the chip surface with 50 mM N-hydroxysuccinimide and 200 mM N-(dimethylaminopropyl)-N'-ethylcarbodiimide, a LeY-BSA stock solution (155 µg/ml) in PBS was diluted with an equal volume of sodium acetate (1 M, pH 3.0) and injected twice to give a high density surface (7000 RU) of immobilized LeY-BSA. For analysis of the MSL5 proteins, 20 µl of each sample was injected at various concentrations (IgM: 0.3 - 14 nM, scFv: 0.37 - 3.75 µM; dimeric mini-antibody: 0.12 - 2.4 µM and multimeric MSL5-p53-His: 0.08 - 1.07 µM). Dissociation was induced by injection of HBS buffer. After each measurement, the chip surface was regenerated with 10 µl of 2 M guanidinium HCl diluted with an equal volume of HBS buffer.

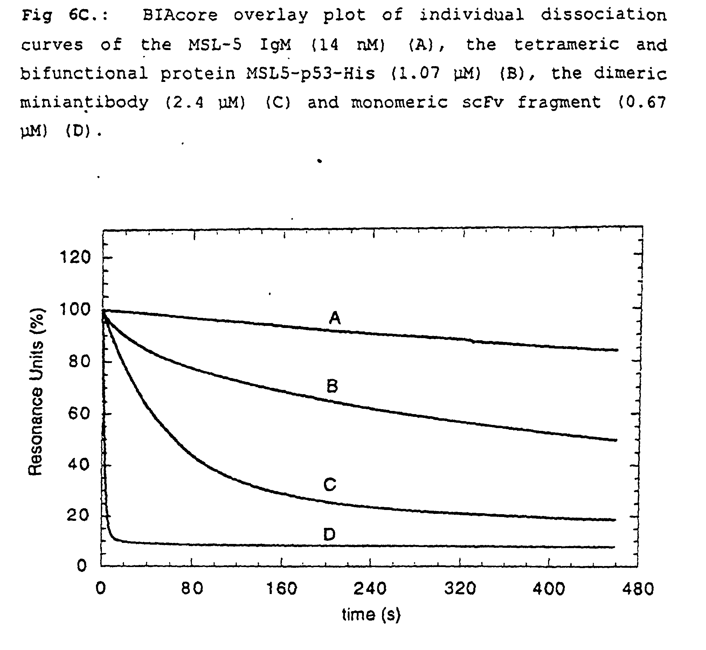

The overlay plot of dissociation curves (Fig. 6c) reveals the second function of the tetrameric complex, namely the binding to a specific target, and illustrates the gain of avidity (functional affinity) by tetramerization of the scFv fragment antibody fragment. Compared to a monovalent scFv fragment and the dimeric construct scdHLX (PCT/EP93/00082), the tetrameric and thus tetravalent protein of this invention (MSL5-p53-His or "multibody") shows a significant enhancement of binding affinity to a surface covered with Le^{Y}-antigens. The gain in avidity can clearly be seen by the prolongation of the dissociation rate from milliseconds (scFv) to several minutes (MSL5-P53-His, Fig 6C).

The modular system of gene cassettes encoding the different association domains allows the facile generation of multivalent and bifunctional proteins with defined valency and steric properties. The multimeric protein of example 1 with four immunoglobulin binding sites can be an ideal design for applications where fast excretion and poor functional affinity of a monovalent fragment does not result in a significant, long-lasting enrichment at the target.

Furthermore, the dependence of the avidity (or "functional affinity") on the actual antigen density (Pack et al., J. Molecular Biology 246, 28-34) will allow a distinction between different tissues having different antigen densities. LeY is found in high densities on colon and breast cancer cells, but in low densities on a variety of normal cells (Sakamoto et al., 1986, Cancer Res. 46, 1553 - 1559). Since antibodies or immunotoxins with higher intrinsic affinity against LeY and related antigens also bind to cell surfaces with low antigen density, significant binding to normal tissues is observed (Trail et al., 1993, Science 261, 212 - 215). In principle, proteins of this invention with high avidity (by the multiplicity of binding events per molecule on a sufficient antigen densitiy) but comparatively low intrinsic affinities (affinity per monovalent interaction, occuring also on low densities) are unable to bind to cells with low densities. Therefore, they will give rise to more selective targeting of cells with over-expressed antigen, which favor multivalent binding and will not cause unwanted side-effects by binding to normal cells with low antigen density.

**Example 2:** Formation of tetrameric complex incorporating four copies anti-phosphocholine scFv antibody fragments and four copies of a a human IL2 by fusion of the two different functional domains to the termini of a tetramerization device based on human p53 via flexible linkers.

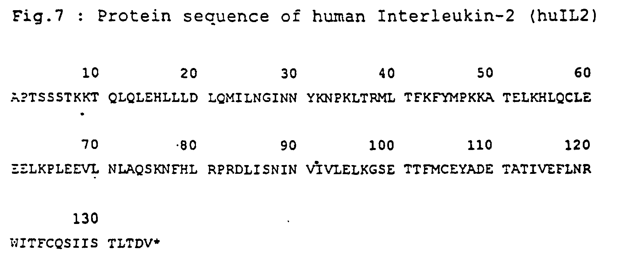

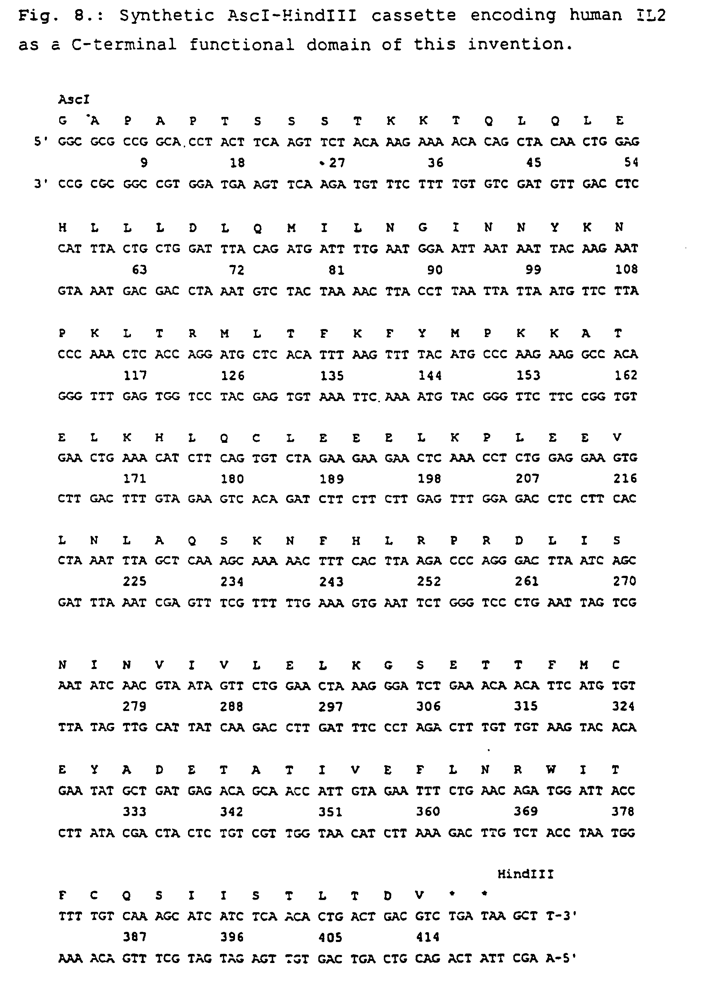

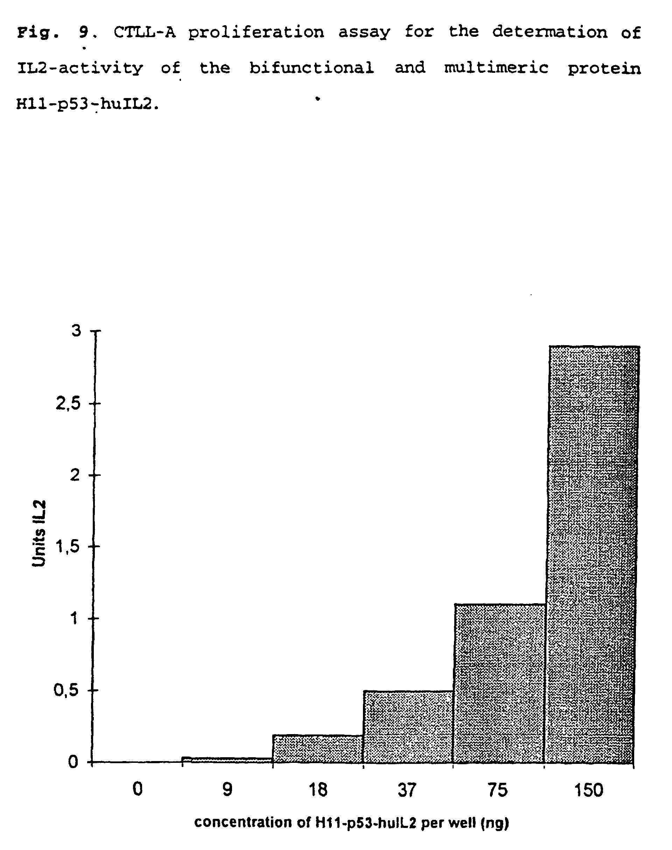

According to example 1, a multimeric fusion protein is encoded by a cistron, which comprise modular gene cassettes encoding a signal sequence, a scFv antibody fragment as a N-terminal functional domain, a first flexible linker based on human IgG3 hinge, a tetramerization domain based on the human p53, a second linker and human IL2 as a C-terminal functional domain (Fig.8,9).

After translation and secretion in a suitable host according to example 1, the multimeric protein H11-p53-huIL2 is purified by the ability of its N-terminal functional domain to bind specifically to the antigen phoshocholine on a affinity column (Glockshuber et al., 1990, Biochemistry 29, 1362-1367). After this purification step, the function of its C-terminal domain, human IL2, was tested in a proliferation assay of cytotoxic T-lympocytes (CTLL-A).

3 x 10⁶ cultivated CTLL-A cells, which can only grow in the presence of functional IL2, are extensively washed with IL2-free phosphate buffer (20 mM NaH₂PO₄, 100 mM NaCl, pH 7.4.) 2.5 % FCS, centrifuged and resuspended in IL2-free standard media with 10 % FCS. Aliquots of aproximately 1.5 x 10⁴ cells in a 50 microliter volume are pipetted into each well of a 96-well microtiter plate. Increasing amounts of recombinant human IL2 for calibration (0.5 - 40 U; Sigma) as well as a dilution series of the phosphocholine-affinity purified H11-p53-huIL2 protein (10 ng - 150 ng) were co-incubated in duplicates for each concentration. After 24h at 37°C, the activity of active mitochondrial dehydrogenase (reflecting the number of living cells) is determined with MTT according to Mossmann (1983, J. Immunological Methods 65, 55-57). The IL2 activity of the C-terminal functional domain of the multimeric protein (Fig. 9) is calculated using the calibration curve.

The proliferation of CTLL-A cells in the presence of the multimeric protein, which was purified with the help of the anti-phosphocholine scFv as the N-terminal functional domain, clearly demonstrates the activity of the huIL2 as the C-terminal functional domain.
The surprising result, that the central multimerization domain allows independent native folding of the distinct domains with subsequent self-assembly, illustrates the potential of this invention.

The central (-linker1-device-linker2-) part of the multimeric protein serves, apart from its multimerization function, as a very long and protease-stable "spacer", allowing independent folding of the considerably (up to 6 times) larger fused domains. A conventional, extremely long linker of the same length as the central (-linkerl-device-linker2-) part but without its secondary structure is probably much more protease-sensitive, as it is typical for unstructured peptides, which are well accessible for the catalytic pocket of proteases (Argos, 1990, J. Mol. Biol. 211, 943-958).

**Example 3:** Formation of tetrameric and bifunctional complex incorporating four copies of anti-ESL-1 scFv antibody fragments and four copies of a detection tag which in addition is used as a metal-binding domain by fusion of the two different functional domains to the termini of a small tetramerization device based on human p53 via flexible linkers.

The multimerization device of this example is described in Example 1.

A cistron of this fusion protein is constructed by the ligation of the following modular gene cassettes (5' -> 3') to a cistron under a lac promoter/operator system in an E. coli expression plasmid according to example 1:
- a XbaI-EcoRV cassette encoding an ompA signal sequence
- an EcoRV-EcoRI cassette encoding ESL-1 binding scFv antibody fragments
- an EcoRI-MroI AscI gene cassette encoding a flexible upper hinge of human IgG3 (Fig. 4)
- a MroI-AscI encoding a multimerization device derived from the human IgG3 residues human p53 and a flexible GGSGGAP linker incorporating the in-frame AscI-site (Fig. 3)
- a AscI-HindIII gene cassette encoding a functional domain for purification purposes which complexes metal ions and detection purposes of being specifically recognized by an antibody (Fig. 5).

The ESL-1 binding scFv antibodies were derived from a human combinatorial antibody library using phage display. After three rounds of panning 12 clones different in the sequence of the CDR3 heavy chain were selected and cloned via XbaI and EcoRI in to an appropriate expression vector (Fig. 6a). The human combinatorial antibody library construction and the panning of the ESL-1 binding antibodies is described in EP application 9511 3021.0-2110 "Protein libraries", the teachings of which are incorporated herein by reference. After transformation of the expression plasmids into an E. coli JM83 strain and induction of the lac promoter with IPTG at 30°C in shaking cultures for 4 h, the translated fusion proteins were secreted into the periplasm as self-associating tetramers (scFv-linker1-device-linker2-purification domain) without the need of refolding or chemical cross-linking. For lysis, the cells (4 ml) were collected by centrifugation, re-dissolved in 800 µl PBS pH 7.4 and sonicated for 90 sec. After another centrifugation step the supernatant was used for Western Blot analysis and binding experiments (ELISA). In parallel, conventional monomeric fusion proteins consisting of scFvs directly fused to the purification domain were prepared accordingly.

For characterizing the amounts of soluble secreted protein a Western Blot analysis was performed. 20 µl of lysed bacterial supernatant was loaded on a 12.5 % polyacryl amide gel. After blotting the gel, the nitrocellulose filter was blocked with 3 % milk powder. In addition to using the metal binding function of the C-terminal domain for purification purposes the C-terminal domain was used as a detection tag specifically recognized by an anti-His antibody (Dianova).

It is demonstrated that, similar to the case with the LeY binding antibody (Example 1), the multimerization device is compatible with secretion and folding of the anti-ESL-1 binding scFv fragments, so that no significant reduction in functional yields is seen in this example as judged from the amounts of soluble protein detected in the Western Blot.

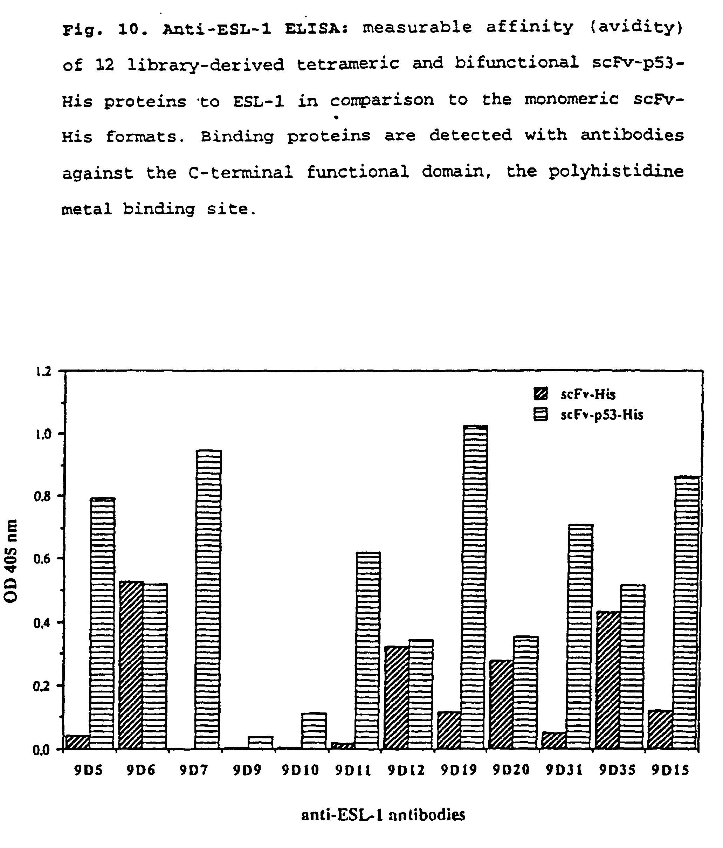

The characterization of binding activity to surface bound ESL-1 reveals the second function of the tetrameric complex, namely the binding to a specific target. For example, in an ELISA experiment, the antigen (ESL-1, 5µg/ml) was coated overnight at room temperature to a microtitre plate. After blocking with 3 % milk powder, the antigen was incubated for 2 h at room temperature with 200 µl cell lysate either expressing monomeric or tetrameric ESL-1 binding antibody fragments fused to the His-detection tag. After washing with PBS pH 7.4 (5x) the detection of ESL-1 binding was assayed by incubation with a first anti-His antibody (Dianova) and a second antibody linked to alkaline phosphatase and directed against the first antibody. Each measurement was performed in duplicate (Fig. 10).

The binding experiments of the ESL-1 binding antibody fragments reveal both functions of the tetrameric complex, namely the binding to a specific target and the detection of bound complexes, and illustrates the gain of avidity by tetramerization of the N-terminal functional domain, namely the scFv fragment antibody fragment. Compared to a monovalent scFv fragment the tetravalent and bifunctional proteins of this invention (each incorporating four copies of anti-ESL-1 scFv fragments) show a significant enhancement of measurable binding activity to a surface covered with ESL-1-antigens up to 10 or more times (9D5, 9D11, 9D31). In some cases, only binding of the multimeric and bifunctional complex of this invention is detectable (9D7, 9D9, 9D10) due to the synergistic effect of multiple binding sites (avidity-effect).

**Example 4:** Hetero-tetramerization device based on parts of the human histones H3 and H4

A synthetic gene containing the genetic information for the part of human histones H3 (Fig. 12a) and H4 (Fig. 12b) responsible for hetero-tetramerization are constructed with suitable restriction sites for 5' and 3' ligations according to Fig. 3, 20 and 22. The H3 and H4 based devices can be used for the hetero-tetramerization of multimeric proteins incorporating up to four different functional domains such as cytokines, toxins, immunoglobulins, enzymes, kinases, phosphatases, lectins, peptide hormones, cell adhesior proteins such as integrins, metal-binding domains, purification devices, in particular peptides which are able to bind to an independent binding entity, peptidic vaccines, bioactive peptides, preferably of 5 to 15 amino acid residues, soluble cell surface proteins such as the CD molecules of leucocytes, DNA binding domains, transcription factors and growth factors. The hetero-tetramerization requires either co-expression of the two fusion proteins (domain1-linker1-device1 (e.g. H3) -linker2-device2 and domain3-linker3-device2 (e.g. H4) -linker4-device4) with the use of a suitable dicistronic expression system (Ge et al., 1995, Antibody Engineering 2nd edition, C.A.K. Borrebaeck, ed. Oxford University Press, pp 229-266) or separate expression with subsequent mixture and in vitro assembly.
Instead of human histones H3 and H4, the hetero-tetramerization device can be based on sequences derived from the human hTAF_{II}31 and hTAF_{II}80 (Fig. 11 a,b).

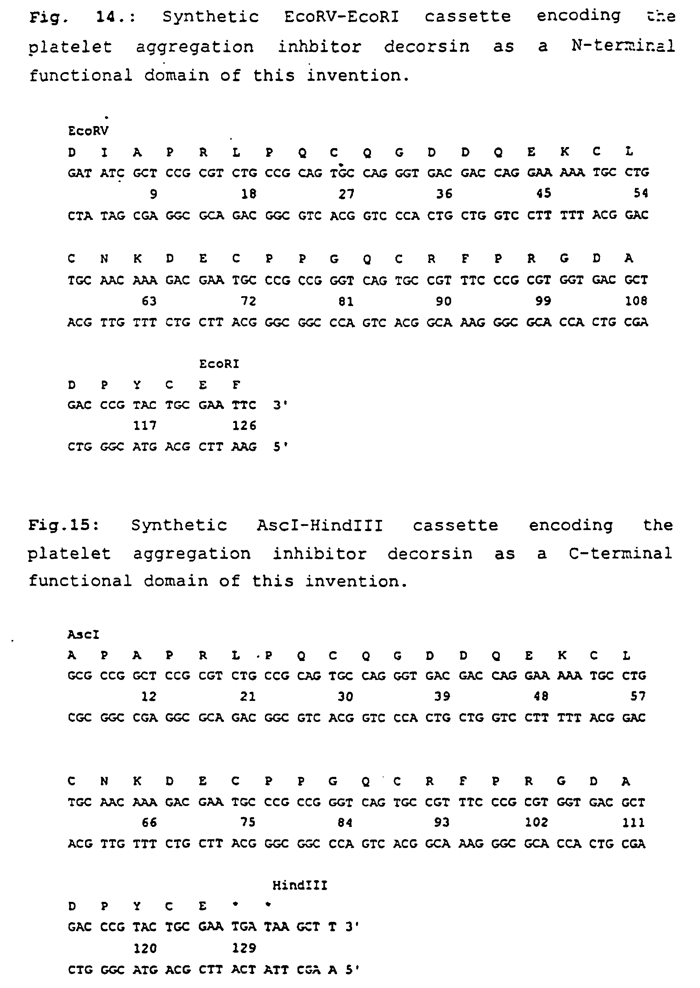

**Example 5:** As a functional domain, the platelet aggregation inhibitor decorsin (Fig. 13) is either encoded by a N-terminal EcoRV-EcoRI (Fig. 14) or as C-terminal AscI-HindIII gene cassette (Fig. 15) and fused to a peptidic multimerization device of this invention via flexible linkers according to examples 1, 2 and 3.

**Example 6:** Formation of octavalent anti-LPS peptides (Fig. 16) by fusion to a tetramerization device based on the human p53.

The bioactive peptides can be fused as a gene cassette with suitable restriction sites either to the N-terminus (Fig. 17) or to the C-terminus (Fig. 18) of the assembly domain via the peptide linkers according to examples 1, 2 and 3.

**Example 7:** Tetramerization device based on the human serum protein PF4.

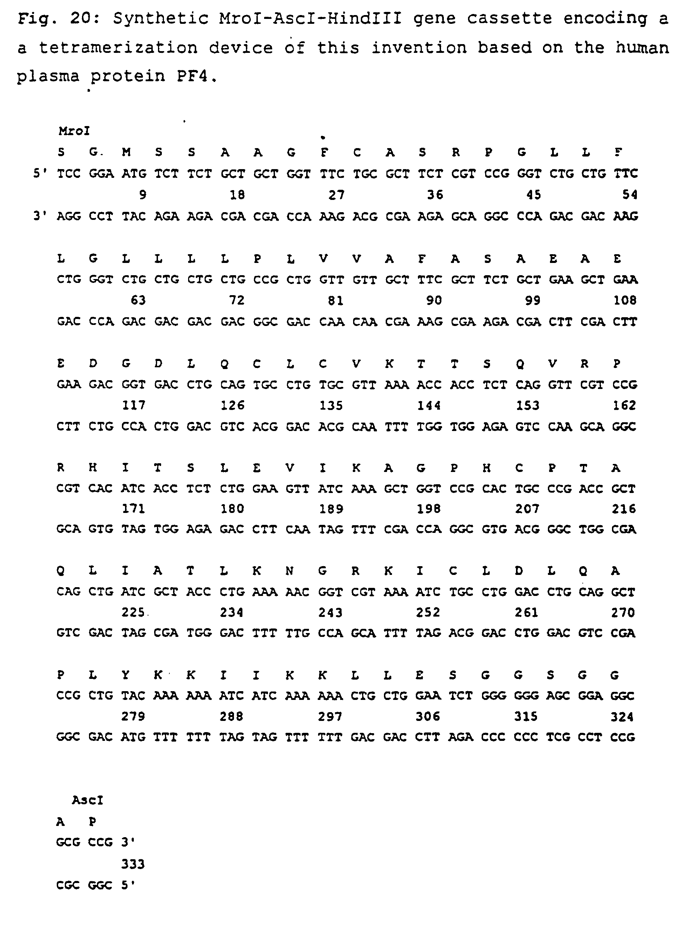

A synthetic gene containing the genetic information for PF4 (Fig. 19) and suitable restriction sites for 5' and 3' ligations (Fig. 20) can be used for the tetramerization of multimeric proteins incorporating functional domains such as cytokines, toxins, immunoglobulins, enzymes, kinases, phosphatases, lectins, peptide hormones, cell adhesion proteins such as integrins, metal-binding domains, purification devices, in particular peptides which are able to bind to an independent binding entity, peptidic vaccines, bioactive peptides, preferably of 5 to 15 amino acid residues, soluble cell surface proteins such as the CD molecules of leucocytes, DNA binding domains, transcription factors and growth factors according to Examples 1 to 3.

**Example 8:** Pentamerization device based on human TSP4 (Fig. 21), which can be used for the pentamerization of multimeric proteins incorporating functional domains such as cytokines, toxins, immunoglobulins, enzymes, kinases, phosphatases, lectins, peptide hormones, cell adhesion proteins such as integrins, metal-binding domains, purification devices, in particular peptides which are able to bind to an independent binding entity, peptidic vaccines, bioactive peptides, preferably of 5 to 15 amino acid residues, soluble cell surface proteins such as the CD molecules of leucocytes, DNA binding domains, transcription factors and growth factors according to Examples 1, 2 and 3.

## Claims

1. A DNA sequence encoding:
a) a first functional domain,
b) a first linker sequence,
c) a peptidic multimerization device of not more than 110 and preferably 30-80 amino acids in length, which is capable of self-assembly to a trimer or higher order oligomer, and which is of mammalian and preferably of human composition,
d) a second linker sequence, and
e) a second functional domain.

2. The DNA sequence according to claim 1 in which said multimerization device comprises at least a part of the human p53 protein.

3. The DNA sequence according to claim 1 or 2 in which said peptidic multimerization device comprises amino acids 319 to 360 of the human protein p53.

4. The DNA sequence according to claim 1 in which said peptidic multimerization device comprises at least a part of the human PF4 protein.

5. The DNA sequence according to claim 1 in which said peptidic multimerization device comprises at least a part of the human TSP-4 protein.

6. The DNA sequence according to claim 1 in which said peptidic multimerization device comprises at least a part of the human COMP protein.

7. The DNA sequence according to claim 1 in which said peptidic multimerization device comprises at least a part of human thrombospondin.

8. The DNA sequence according to claim 1 in which said peptidic multimerization device comprises at least part of hTAF_{II}31.

9. The DNA sequence according to claim 1 in which said peptidic multimerization device comprises at least part of hTAF_{II}80.

10. The DNA sequence according to claim 1 in which said peptidic multimerization device comprises at least part of histone 3, preferably human histone 3.

11. The DNA sequence according to claim 1 in which said peptidic multimerization device comprises at least part of histone 4, preferably human histone 4.

12. The DNA sequence of any one of claims 1 to 11 encoding appropriately designed additional cysteines as part of the multimerization device to allow for intermolecular covalent linkage of the multimerization device during or after self-assembly.

13. The DNA sequence of any one of claims 1 to 12 encoding appropriately designed additional cysteines as part of the multimerization device or the linker(s) to allow for intermolecular covalent linkage to additional functional domains, which are separately produced in vivo or in vitro and coupled to the multimeric protein via oxidation of a free cystein to result in a covalent disulphide bridge.

14. The DNA sequence according to any one of claims 1 to 13
wherein said linker sequences b) and d) are the same.

15. The DNA sequence according to any one of claims 1 to 13
wherein said linker sequences b) and d) are different.

16. The DNA sequence according to any of claims 1-15 in which said linkers are from the human inter-domain linking sequence.

17. The DNA sequence according to claim 16 in which said human inter-domain linking sequence comprises at least a part of a human antibody hinge sequence.

18. The DNA sequence according to claim 17 in which said human inter-domain linking sequence comprises at least a part of a human IgG3 hinge sequence.

19. The DNA sequence according to any of claims 1-18 in which one or both of said functional domains either:
a) bind to a defined target substance, or
b) catalyze reaction of a defined substrate, or
c) inhibit the action of an enzyme, or
d) bind or block a receptor binding site, or
e) bind to a metal ion.

20. The DNA sequence according to claim 19 in which one or both of said functional domains comprise at least a part of a member of the immunoglobulin super-family.

21. The DNA sequence according to claim 20 in which one or both of said functional domains is a single chain Fv-fragment.

22. The DNA sequence according to claim 20 in which one or both of said members of the immunoglobulin super-family binds to a carbohydrate target substance.

23. The DNA sequence according to claim 22 in which one or both of said members of the immunoglobulin super-family binds to Lewis Y-related carbohydrate structures.

24. The DNA sequence according to any one of claims 1 to 23 wherein said multimerization device is derived from a randomized DNA library in a suitable host, from which DNA members are identified by their ability to encode multimerizing peptides.

25. A vector, preferably an expression vector, comprising a DNA sequence according to any of claims 1-24.

26. The vector according to claim 25 comprising a first DNA sequence encoding a signal sequence for bacterial secretion and a second DNA sequence according to any of claims 1-24.

27. A host cell comprising at least one vector according to claim 25 or 26.

28. The host cell according to claim 27 which is mammalian, preferably human, yeast, plant, insect, preferably Spodoptera frugiperda or bacterial, preferably an E. coli cell.

29. A protein, preferably a fusion protein, comprising:
a) a first functional domain,
b) a first linker sequence,
c) a peptidic multimerization device of not more than 110 and preferably 30-80 amino acid residues in length, which is capable of self-assembly to a trimer or higher order oligomer, and which is of mammalian, preferably human composition,
d) a second linker sequence, and
e) a second functional domain.

30. A fusion protein encoded by the DNA sequence of any one of claims 1 to 24, the vector of any one of claims 25 to 26 or/and produced by the cellular host of claim 27 or 28.

31. A multimeric protein assembled from proteins and/or fusion proteins according to claim 29 or 30.

32. The multimeric protein of claim 31 which is a homomultimeric protein.

33. The multimeric protein of claim 31 which is a heteromultimeric protein.

34. A method of producing the fusion protein according to claim 29 or 30, said method comprising growing a cellular host according to claim 27 or 28 in an appropriate medium whereby said fusion protein is expressed, and isolating said fusion protein.

35. A method of producing a fusion protein according to claim 29 or 30, said method comprising growing a cellular host according to claim 27 or 28 in an appropriate medium whereby said protein is expressed, and secreted to the periplasm of said host, and isolating said protein, preferably as a multimeric protein from said periplasm.

36. A method for producing a protein comprising
a) a first functional domain,
b) a first linker sequence,
c) a multimerization device of not more than 110 and preferably 30-80 amino acids in length, which is capable of self-assembly to a trimer or higher order oligomer, and which is of mammalian and preferably human composition,
d) a second linker sequence, and
e) a second functional domain,
wherein at least one of a) to e) is
i) produced recombinantly, and, if all of a) to e) are produced recombinantly, at least two of the constituents are produced by different host cells; and/or
ii) produced synthetically; and/or
iii) produced semisynthetically; and/or
iv) produced by in-vitro translation; and
wherein at least two of a) to e) are combined by enzymatic and/or chemical coupling thereby giving rise to the complete protein, and preferably to the complete multimeric protein.

37. A method of producing a multimeric protein, said method comprising associating at least three proteins according to claim 36, said method giving rise either to homomultimeric or heteromultimeric proteins.

38. A diagnostic composition comprising at least a fusion protein according to claim 29 or 30, and/or a multimeric protein according to any one of claims 31 to 33.

39. A pharmaceutical composition comprising at least a fusion protein according to claim 29 or 30, and/or a multimeric protein according to any one of claims 31 to 33 optionally in combination with a pharmaceutically acceptable carrier.

40. A gene cassette comprising at least one DNA sequence according to any one of claims 1 to 24.

41. A kit comprising at least one gene cassette according to claim 40.

## Patentansprüche

1. DNA-Sequenz, codierend:
(a) eine erste funktionelle Domäne,
(b) eine erste Linkersequenz,
(c) ein peptidisches Multimerisierungsmittel mit einer Länge von nicht mehr als 110 und vorzugsweise 30-80 Aminosäuren, das zum Selbstzusammenbau in ein Trimer oder ein Oligomer höherer Ordnung befähigt ist, und das von Säuger- und vorzugsweise von menschlicher Zusammensetzung ist,
(d) eine zweite Linkersequenz, und
(e) eine zweite funktionelle Domäne.

2. DNA-Sequenz nach Anspruch 1, in der das Multimerisierungsmittel zumindest einen Teil des menschlichen p53-Proteins umfasst.

3. DNA-Sequenz nach Anspruch 1 oder 2, in der das peptidische Multimerisierungsmittel die Aminosäuren 319 bis 360 des menschlichen p53-Proteins umfasst.

4. DNA-Sequenz nach Anspruch 1, in der das peptidische Multimerisierungsmittel zumindest einen Teil des menschlichen PF4-Proteins umfasst.

5. DNA-Sequenz nach Anspruch 1, in der das peptidische Multimerisierungsmittel zumindest einen Teil des menschlichen TSP-4-Proteins umfasst.

6. DNA-Sequenz nach Anspruch 1, in der das peptidische Multimerisierungsmittel zumindest einen Teil des menschlichen COMP-Proteins umfasst.

7. DNA-Sequenz nach Anspruch 1, in der das peptidische Multimerisierungsmittel zumindest einen Teil des menschlichen Thrombospondins umfasst.

8. DNA-Sequenz nach Anspruch 1, in der das peptidische Multimerisierungsmittel zumindest einen Teil des hTAF_{∥}31 umfasst.

9. DNA-Sequenz nach Anspruch 1, in der das peptidische Multimerisierungsmittel zumindest einen Teil des hTAF_{∥}80 umfasst.

10. DNA-Sequenz nach Anspruch 1, in der das peptidische Multimerisierungsmittel zumindest einen Teil von Histon 3, vorzugsweise menschliches Histon 3, umfasst.

11. DNA-Sequenz nach Anspruch 1, in der das peptidische Multimerisierungsmittel zumindest einen Teil von Histon 4, vorzugsweise menschliches Histon 4, umfasst.

12. DNA-Sequenz nach einem der Ansprüche 1 bis 11, codierend für passend gestaltete, zusätzliche Cysteine als Teil des Multimerisierungsmittels, um eine intermolekulare kovalente Verknüpfung des Multimerisierungsmittels während oder nach dem Selbstzusammenbau zu erlauben.

13. DNA-Sequenz nach einem der Ansprüche 1 bis 12, codierend für passend gestaltete, zusätzliche Cysteine als Teil des Multimerisierungsmittels oder der Verknüpfung(en), um eine intermolekulare kovalente Verknüpfung mit zusätzlichen funktionellen Domänen zu erlauben, die separat in vivo oder in vitro produziert werden und an das multimere Protein über Oxidation eines freien Cysteins gekoppelt werden, so dass eine kovalente Disulfidbrücke entsteht.

14. DNA-Sequenz nach einem der Ansprüche 1 bis 13, wobei die Verknüpfungssequenzen b) und d) die gleichen sind.

15. DNA-Sequenz nach einem der Ansprüche 1 bis 13, wobei die Verknüpfungssequenzen b) und d) unterschiedlich sind.

16. DNA-Sequenz nach einem der Ansprüche 1 bis 15, in der die Linker von der menschlichen Interdomänenlinkersequenz sind.

17. DNA-Sequenz nach Anspruch 16, in der die menschliche Interdomänenlinkersequenz zumindest einen Teil einer menschlichen Antikörpergelenksequenz umfasst.

18. DNA-Sequenz nach Anspruch 17, in der die menschliche Interdomänenlinkersequenz zumindest einen Teil einer menschlichen IgG3-Gelenksequenz umfasst.

19. DNA-Sequenz nach einem der Ansprüche 1 bis 18, in der eine oder beide der funktionellen Domänen entweder:
a) an eine definierte Zielsubstanz binden, oder
b) die Umsetzung eines definierten Substrates katalysieren, oder
c) die Wirkung eines Enzyms hemmen, oder
d) eine Rezeptorbindungsstelle binden oder blockieren, oder
e) an ein Metallion binden.

20. DNA-Sequenz nach Anspruch 19, in der eine oder beide der funktionellen Domänen zumindest einen Teil eines Mitglieds der Immunglobulin-Superfamilie umfassen.

21. DNA-Sequenz nach Anspruch 20, in der eine oder beide der funktionellen Domänen ein Einzelketten-Fv-Fragment ist.

22. DNA-Sequenz nach Anspruch 20, in der eine oder beide der Mitglieder der Immunglobulin-Superfamilie an eine Kohlenhydrat-Zielsubstanz bindet.

23. DNA-Sequenz nach Anspruch 22, in der eine oder beide der Mitglieder der Immunglobulin-Superfamilie an Lewis-Y-verwandte Kohlenhydratstrukturen bindet.

24. DNA-Sequenz nach einem der Ansprüche 1 bis 23, wobei das Multimerisierungsmittel von einer randomisierten DNA-Bibliothek in einem geeigneten Wirt abgeleitet ist, aus der DNA-Mitglieder durch ihre Fähigkeit, multimerisierende Peptide zu codieren, identifiziert werden.

25. Vektor, vorzugsweise ein Expressionsvektor, umfassend eine DNA-Sequenz nach einem der Ansprüche 1 bis 24.

26. Vektor nach Anspruch 25, umfassend eine erste DNA-Sequenz, die eine Signalsequenz für bakterielle Sekretion codiert, und eine zweite DNA-Sequenz nach einem der Ansprüche 1 bis 24.

27. Wirtszelle, umfassend zumindest einen Vektor nach Anspruch 25 oder 26.

28. Wirtszelle nach Anspruch 27, die von einem Säuger, vorzugsweise Mensch, Hefe, Pflanze, Insekt, vorzugsweise Spodoptera frugiperda, oder bakteriell, vorzugsweise eine E. coli-Zelle, ist.

29. Protein, vorzugsweise ein Fusionsprotein, umfassend:
a) eine erste funktionelle Domäne,
b) eine erste Linkersequenz,
c) ein peptidisches Multimerisierungsmittel mit einer Länge von nicht mehr als 110 und vorzugsweise 30-80 Aminosäureresten, das zum Selbstzusammenbau in ein Trimer oder ein Oligomer höherer Ordnung befähigt ist, und das von Säuger-, vorzugsweise menschlicher Zusammensetzung ist,
d) eine zweite Linkersequenz, und
e) eine zweite funktionelle Domäne.

30. Fusionsprotein, das von der DNA-Sequenz nach einem der Ansprüche 1 bis 24 codiert wird, dem Vektor nach einem der Ansprüche 25 bis 26 oder/und das durch den zellulären Wirt von Anspruch 27 oder 28 hergestellt wird.

31. Multimeres Protein, das aus den Proteinen und/oder Fusionsproteinen nach Anspruch 29 oder 30 zusammengebaut ist.

32. Multimeres Protein nach Anspruch 31, das ein homomultimeres Protein ist.

33. Multimeres Protein nach Anspruch 31, das ein heteromultimeres Protein ist.

34. Verfahren zur Herstellung des Fusionsproteins nach Anspruch 29 oder 30, wobei das Verfahren das Züchten eines zellulären Wirtes nach Anspruch 27 oder 28 in einem passenden Medium umfasst, wodurch das Fusionsprotein exprimiert wird, und das Isolieren des Fusionsproteins.

35. Verfahren zur Herstellung eines Fusionsproteins nach Anspruch 29 oder 30, wobei das Verfahren das Züchten eines zellulären Wirtes nach Anspruch 27 oder 28 in einem passenden Medium umfasst, wodurch das Protein exprimiert und in das Periplasma des Wirtes sekretiert wird, und das Isolieren des Proteins aus dem Periplasma, vorzugsweise als ein multimeres Protein.

36. Verfahren zur Herstellung eines Proteins, umfassend:
a) eine erste funktionelle Domäne,
b) eine erste Linkersequenz,
c) ein Multimerisierungsmittel mit einer Länge von nicht mehr als 110 und vorzugsweise 30-80 Aminosäureresten, das zum Selbstzusammenbau in ein Trimer oder ein Oligomer höherer Ordnung befähigt ist, und das von Säuger-, vorzugsweise menschlicher Zusammensetzung ist,
d) eine zweite Linkersequenz, und
e) eine zweite funktionelle Domäne,
wobei zumindest eine von a) bis e)
i) rekombinant hergestellt, und, falls alle von a) bis e) rekombinant hergestellt werden, zumindest zwei der Bestandteile von unterschiedlichen Wirtszellen hergestellt werden; und/oder
ii) synthetisch hergestellt; und/oder
iii) halbsynthetisch hergestellt; und/oder
iv) durch in vitro-Translation hergestellt wird; und
wobei zumindest zwei von a) bis e) durch enzymatische und/oder chemische Kopplung kombiniert werden, so dass das vollständige Protein, und vorzugsweise das vollständige multimere Protein entsteht.

37. Verfahren zur Herstellung eines multimeren Proteins, wobei das Verfahren das Assoziieren von zumindest drei Proteinen nach Anspruch 36 umfasst, wobei das Verfahren entweder ein homomultimeres oder ein heteromultimeres Protein entstehen lässt.

38. Diagnostikmittel, umfassend zumindest ein Fusionsprotein nach Anspruch 29 oder 30 und/oder ein multimeres Protein nach einem der Ansprüche 31 bis 33.

39. Arzneimittel, umfassend zumindest ein Protein nach Anspruch 29 oder 30, und/oder ein multimeres Protein nach einem der Ansprüche 31 bis 33 gegebenenfalls in Kombination mit einem pharmazeutisch verträglichen Träger.

40. Genkassette, umfassend zumindest eine DNA-Sequenz nach einem der Ansprüche 1 bis 24.

41. Kit, umfassend zumindest eine Genkassette nach Anspruch 40.

## Revendications

1. Séquence d'ADN codant pour :
a) un premier domaine fonctionnel,
b) une première séquence de maillon,
c) un bloc de multimérisation peptidique de pas plus de 110 et de préférence de 30 à 80 acides aminés en longueur, lequel est capable d'auto-assemblage à un trimère ou à un oligomère de plus grand ordre, et qui est de composition mammifère et de préférence humaine,
d) une deuxième séquence de maillon,
e) un deuxième domaine fonctionnel.

2. Séquence d'ADN selon la revendication 1, dans laquelle ledit bloc de multimérisation comprend au moins une partie de la protéine humaine p53.

3. Séquence d'ADN selon l'une des revendications 1 ou 2, dans laquelle ledit bloc de multimérisation peptidique comprend les acides aminés 319 à 360 de la protéine humaine p53.

4. Séquence d'ADN selon la revendication 1, dans laquelle ledit bloc de multimérisation peptidique comprend au moins une partie de la protéine humaine PF4.

5. Séquence d'ADN selon la revendication 1, dans laquelle ledit bloc de multimérisation peptidique comprend au moins une partie de la protéine humaine TSP-4.

6. Séquence d'ADN selon la revendication 1, dans laquelle ledit bloc de multimérisation peptidique comprend au moins une partie de la protéine humaine COMP.

7. Séquence d'ADN selon la revendication 1, dans laquelle ledit bloc de multimérisation peptidique comprend au moins une partie de la thrombospondine humaine.

8. Séquence d'ADN selon la revendication 1, dans laquelle ledit bloc de multimérisation peptidique comprend au moins une partie de la hTAF_{∥}31.

9. Séquence d'ADN selon la revendication 1, dans laquelle ledit bloc de multimérisation peptidique comprend au moins une partie de la hTAF_{∥}80.

10. Séquence d'ADN selon la revendication 1, dans laquelle ledit bloc de multimérisation peptidique comprend au moins une partie de l'histone 3, de préférence de l'histone 3 humaine.

11. Séquence d'ADN selon la revendication 1, dans laquelle ledit bloc de multimérisation peptidique comprend au moins une partie de l'histone 4, de préférence de l'histone 4 humaine.

12. Séquence d'ADN selon l'une quelconque des revendications 1 à 11, codant pour des cystéines additionnelles conçues de manière appropriée en tant que parties du bloc de multimérisation pour permettre un accrochage covalent intermoléculaire du bloc de multimérisation pendant ou après l'auto-assemblage.

13. Séquence d'ADN selon l'une quelconque des revendications 1 à 12, codant pour des cystéines additionnelles conçues de manière appropriée en tant que parties du bloc de multimérisation ou en tant que maillon(s) pour permettre un accrochage covalent intermoléculaire à des domaines fonctionnels additionnels, lesquels sont produits séparément in vivo ou in vitro et couplés à la protéine multimère au moyen d'une oxydation d'une cystéine libre, ce qui a pour résultat un pont disulfure covalent.

14. Séquence d'ADN selon l'une quelconque des revendications 1 à 13, dans laquelle lesdites séquences de maillon b) et d) sont les mêmes.

15. Séquence d'ADN selon l'une quelconque des revendications 1 à 13, dans laquelle lesdites séquences de maillon b) et d) sont différentes.

16. Séquence d'ADN selon l'une quelconque des revendications 1 à 15, dans laquelle lesdits maillons proviennent d'une séquence d'accrochage inter-domaine humaine.

17. Séquence d'ADN selon la revendication 16, dans laquelle ladite séquence d'accrochage inter-domaine humaine comprend au moins une partie d'une séquence charnière d'un anticorps humain.

18. Séquence d'ADN selon la revendication 17, dans laquelle ladite séquence d'accrochage inter-domaine humaine comprend au moins une partie d'une séquence charnière d'un IgG3 humain.

19. Séquence d'ADN selon l'une quelconque des revendications 1 à 18, dans laquelle l'un ou les deux domaines fonctionnels susdits, soit:
a) se lient à une substance cible définie, ou
b) catalysent une réaction d'un substrat défini, ou
c) inhibent l'action d'une enzyme, ou
d) se lient à ou bloquent un côté de liaison à un récepteur, ou
e) se lient à un ion métallique.

20. Séquence d'ADN selon la revendication 19, dans laquelle l'un ou les deux domaines fonctionnels susdits comprennent au moins une partie d'un membre de la superfamille des immunoglobulines.

21. Séquence d'ADN selon la revendication 20, dans laquelle l'un ou les deux domaines fonctionnels susdits sont un fragment Fv d'une chaîne simple.

22. Séquence d'ADN selon la revendication 20, dans laquelle l'un ou les deux membres susdits de la superfamille des immunoglobulines se lie(nt) à une substance cible à base d'un hydrate de carbone.

23. Séquence d'ADN selon la revendication 22, dans laquelle l'un ou les deux membres susdits de la superfamille des immunoglobulines se lie(nt) à des structures d'hydrate de carbone en système Lewis Y.

24. Séquence d'ADN selon l'une quelconque des revendications 1 à 23, dans laquelle ledit bloc de multimérisation provient d'une banque d'ADN aléatoire dans un hôte adéquat, duquel les membres de l'ADN sont identifiés par leur capacité à coder pour des peptides de multimérisation.

25. Vecteur, de préférence un vecteur d'expression, comprenant une séquence d'ADN selon l'une quelconque des revendications 1 à 24.

26. Vecteur selon la revendication 25, comprenant une première séquence d'ADN codant pour une séquence signal de sécrétion bactérienne et une deuxième séquence d'ADN selon l'une quelconque des revendications 1 à 24.

27. Cellule hôte comprenant au moins un vecteur selon la revendication 25 ou 26.

28. Cellule hôte selon la revendication 27, laquelle provient d'un mammifère, de préférence d'un humain, d'une levure, d'une plante, d'un insecte, de préférence de Spodoptera frugiperda ou d'une bactérie, de préférence d'une cellule d'E. coli.

29. Protéine, de préférence une protéine fusion, comprenant :
a) un premier domaine fonctionnel,
b) une première séquence de maillon,
c) un bloc de multimérisation peptidique de pas plus de 110 et de préférence de 30 à 80 résidus d'acides aminés en longueur, lequel est capable d'auto-assemblage à un trimère ou à un oligomère de plus grand ordre, et qui est de composition mammifère et de préférence humaine,
d) une deuxième séquence de maillon,
e) un deuxième domaine fonctionnel.

30. Protéine fusion codée par la séquence d'ADN selon l'une quelconque des revendications 1 à 24, le vecteur selon l'une quelconque des revendications 25 à 26, et/ou produite par l'hôte cellulaire selon la revendication 27 ou 28.

31. Protéine multimère assemblée à partir des protéines et/ou des protéines fusion selon la revendication 29 ou 30.

32. Protéine multimère selon la revendication 31, laquelle protéine est une protéine homomultimère.

33. Protéine multimère selon la revendication 31, laquelle protéine est une protéine hétéromultimère.

34. Procédé de production d'une protéine fusion selon la revendication 29 ou 30, ledit procédé comprenant une croissance d'un hôte cellulaire selon la revendication 27 ou 28 dans un milieu approprié grâce auquel ladite protéine fusion est exprimée, et l'isolation de ladite protéine fusion.

35. Procédé de production d'une protéine fusion selon la revendication 29 ou 30, ledit procédé comprenant une croissance d'un hôte cellulaire selon la revendication 27 ou 28 dans un milieu approprié grâce auquel ladite protéine est exprimée, et sécrété vers le périplasme dudit hôte, et l'isolation de ladite protéine, de préférence sous la forme d'une protéine multimère, à partir dudit périplasme.

36. Procédé pour la production d'une protéine, comprenant :
a) un premier domaine fonctionnel,
b) une première séquence de maillon,
c) un bloc de multimérisation peptidique de pas plus de 110 et de préférence de 30 à 80 résidus d'acides aminés en longueur, lequel est capable d'auto-assemblage à un trimère ou à un oligomère de plus grand ordre, et qui est de composition mammifère et de préférence humaine,
d) une deuxième séquence de maillon,
e) un deuxième domaine fonctionnel,
dans lequel au moins l'un de a) à e) est
i) produit de manière recombinante et, si tous les a) à e) sont produits de manière recombinante, au moins deux des constituants sont produits par des hôtes cellulaires différents, et/ou
ii) produit synthétiquement, et/ou
iii) produit semi-synthétiquement, et/ou
iv) produit par traduction in vitro, et
dans lequel au moins deux des a) à e) sont combinés par couplage enzymatique et/ou chimique pour donner de cette manière naissance à la protéine complète, et de préférence à la protéine multimère complète.

37. Procédé de production d'une protéine multimère, ledit procédé comprenant l'association d'au moins trois protéines selon la revendication 36, ledit procédé donnant naissance à des protéines soit homomultimères soit hétéromultimères.

38. Composition diagnostique comprenant au moins une protéine fusion selon la revendication 29 ou 30, et/ou une protéine multimère selon l'une quelconque des revendications 31 à 33.

39. Composition pharmaceutique comprenant au moins une protéine fusion selon la revendication 29 ou 30, et/ou une protéine multimère selon l'une quelconque des revendications 31 à 33, éventuellement en combinaison avec un excipient pharmaceutiquement acceptable.

40. Cassette de gène comprenant au moins une séquence d'ADN selon l'une quelconque des revendications 1 à 24.

41. Trousse comprenant au moins une cassette de gène selon la revendication 40.
